(19) Europäisches Patentamt
European Patent Office
Office européen des brevets

(11) EP 2 888 314 B1

(12) **EUROPEAN PATENT SPECIFICATION**

(45) Date of publication and mention of the grant of the patent:
**29.06.2016 Bulletin 2016/26**

(21) Application number: **13748116.4**

(22) Date of filing: **23.07.2013**

(51) Int Cl.:
*C08J 3/12* (2006.01) *A61K 8/24* (2006.01)
*A61K 8/55* (2006.01) *A61Q 1/02* (2006.01)
*A61Q 1/10* (2006.01) *A61Q 1/12* (2006.01)
*A61Q 17/04* (2006.01)

(86) International application number:
**PCT/JP2013/070404**

(87) International publication number:
**WO 2014/030497 (27.02.2014 Gazette 2014/09)**

(54) **COMPOSITE PARTICLE AND A COSMETIC COMPOSITION CONTAINING THE SAME**

VERBUNDPARTIKEL UND DIESE ENTHALTENDE KOSMETISCHE ZUSAMMENSETZUNG

PARTICULE COMPOSITE ET SA COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.08.2012 JP 2012182507**

(43) Date of publication of application:
**01.07.2015 Bulletin 2015/27**

(73) Proprietor: **Chanel Parfums Beauté**
**92200 Neuilly-sur-Seine (FR)**

(72) Inventors:
- **ITO, Hisao**
**Funabashi-shi**
**Chiba 273-0045 (JP)**
- **YAMAKI, Hideyuki**
**Funabashi-shi**
**Chiba 273-0045 (JP)**
- **KUROMIYA, Tomomi**
**Funabashi-shi**
**Chiba 273-0045 (JP)**
- **NAGAHIRO, Chikako**
**Funabashi-shi**
**Chiba 273-0045 (JP)**
- **ISHIMORI, Fumitaka**
**Koka-shi**
**Shiga 528-0056 (JP)**

(74) Representative: **Cabinet Plasseraud**
**66, rue de la Chaussée d'Antin**
**75440 Paris Cedex 09 (FR)**

(56) References cited:
- **DATABASE WPI Week 201077 Thomson Scientific, London, GB; AN 2010-N52307 XP002715102, & JP 2010 241785 A (SEKISUI PLASTICS CO LTD) 28 October 2010 (2010-10-28) cited in the application**
- **DATABASE WPI Week 199436 Thomson Scientific, London, GB; AN 1994-290923 XP002715103, & JP H06 220110 A (SEKISUI PLASTICS CO LTD) 9 August 1994 (1994-08-09)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a composite particle and a cosmetic composition containing the same. In more detail, the present invention relates to a composite particle giving an excellent feeling upon application and capable of selectively adsorbing free fatty acid in sebum, and a cosmetic composition containing the composite particle.

### Background Art

There are problems that make-up smudges caused by secretion of sweat and sebum, etc., cause skin dullness, oily shine, and blotchy make-up, etc. Conventionally, an attempt has been made to address such problems by blending a powder having a sebum-absorbing action. As a sebum-absorbing powder, for example, a powder of a spherical porous resin such as calcium phosphate, aluminum magnesium silicate, a vegetable powder, a methacrylic acid ester, and an acrylic acid ester; and a porous powder such as kaolin, mica, talc, crystalline cellulose, and calcium carbonate are known.
Meanwhile, when the application involves direct contact to skin such as a foundation and a sunscreen, such a cosmetic is required to impart a soft and smooth feel to skin upon application.
As a technology that accomplishes both sebum-adsorption performance and a soft and smooth feeling upon application, for example, Japanese Patent Laid-Open No. 2010-241785 (Patent Literature 1) discloses a core-shell-type composite particle comprised of a spherical resin particle, the surface of which is coated with amorphous calcium phosphate such as hydroxyapatite. This patent literature describes that a resin particle as a core particle gives a soft and smooth feel, and amorphous calcium phosphate that is coating the surface thereof like a film effectively adsorbs sebum, thereby suppressing make-up smudges caused by sebum, and moreover, realizes soft focus (blurry image) by light scattering.
Meanwhile, it has recently been reported that unsaturated free fatty acid contained in sebum is the cause of prominent pores (Fragrance Journal 2004-3, pages 41 to 47). It is assumed that if unsaturated free fatty acid in sebum can be effectively adsorbed, then not only make-up smudges can be suppressed, but also a toning effect on the skin texture can be anticipated, which is assumed to further increase the utility of a composite particle in cosmetic use.

### Citation List

#### Patent Literature

Patent Literature 1: Japanese Patent Laid-Open No. 2010-241785

#### Non Patent Literature

Non Patent Literature 1; Fragrance Journal 2004-3, pages 41 to 47

### Summary of Invention

#### Technical Problem

Under the foregoing circumstances, with a view to further enriching the added value of a composite particle in cosmetic use, the provision of a composite particle giving an excellent feeling upon application and capable of effectively preventing make-up smudges, and further desirably, capable of selectively absorbing free fatty acid in sebum, particularly, unsaturated free fatty acid, is demanded. Also, a cosmetic composition containing such a composite particle is demanded.

#### Solution to Problem

The present inventors conducted an intensive research to solve the aforementioned problem. As a result, they have found that the use of a core-shell-type composite particle comprised of a resin particle, the surface of which is coated with amorphous calcium phosphate and calcium carbonate, cannot only accomplish both the sebum adsorption performance and a soft and smooth feeling upon application, but also remarkably increase the absorptivity of free fatty acid, compared to a case in which amorphous calcium phosphate and calcium carbonate are each used independently, thereby completing the present invention.
That is, the present invention relates to the following composite particle and a cosmetic composition containing the composite particle.

[1] A core-shell-type composite particle comprising a resin particle as a core particle having, formed on the surface thereof, a coating layer comprising amorphous calcium phosphate and calcium carbonate,
wherein the resin particle contains a polymer of a vinyl monomer and any one of the following components:

a phosphorous acid diester represented by the following general formula (1):

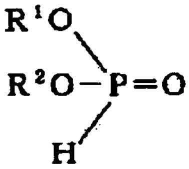

· · · (1)

and a partially esterified phosphoric acid represented by the following general formula (2) or (3):

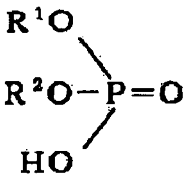

· · · (2)

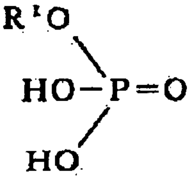

· · · (3)

wherein, $R^1$ and $R^2$ in the general formulae (1) to (3) are each independently a monovalent group formed by removal of one hydrogen atom from an acyclic saturated or unsaturated hydrocarbon having 1 to 14 carbon atoms, or a group represented by the following formula (4):

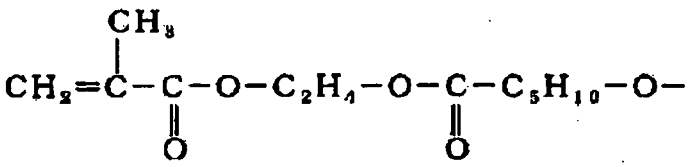

· · · (4)

[2] The composite particle according to [1], wherein a weight ratio of calcium phosphate is 5 to 30 parts by weight relative to 100 parts by weight of the resin particle, and a weight ratio of calcium carbonate is 3 to 20 parts by weight relative to 100 parts by weight of the resin particle.
[3] The composite particle according to [1] or [2], wherein an average particle diameter of the composite particle is 1 to 100 μm.
[4] The composite particle according to any one of [1] to [3], wherein an average particle diameter of calcium carbonate is 0.1 to 0.5 μm.
[5] A cosmetic composition comprising: a core-shell-type composite particle comprising a resin particle as a core particle having, formed on the surface thereof, a coating layer comprising amorphous calcium phosphate and calcium carbonate; and a cosmetically acceptable component,
wherein the resin particle contains a polymer of a vinyl monomer and contains any one of the following components:

a phosphorous acid diester represented by the following general formula (1):

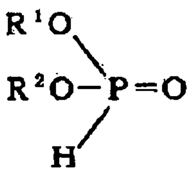

· · · (1)

and a partially esterified phosphoric acid represented by the following general formula (2) or (3):

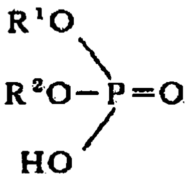 · · · (2) 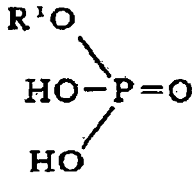 · · · (3)

wherein, $R^1$ and $R^2$ in the general formulae (1) to (3) are each independently a monovalent group formed by removal of one hydrogen atom from an acyclic saturated or unsaturated hydrocarbon having 1 to 14 carbon atoms, or a group represented by the following formula (4):

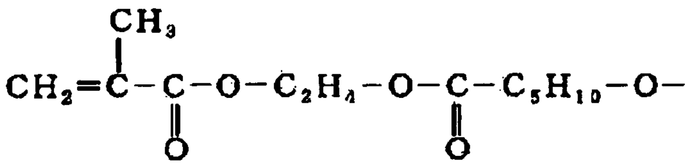 · · · (4)

[6] The cosmetic composition according to [5], wherein the cosmetic composition comprises the composite particle in an amount of 0.1 to 40% by weight of a total weight of the cosmetic composition.
[7] The cosmetic composition according to [5] or [6], wherein the cosmetic composition is a make-up cosmetic or a skin care cosmetic.
[8] The cosmetic composition according to [7], wherein the cosmetic composition is a powder foundation, a W/Si type foundation or O/W type foundation, a loose powder, an eye shadow, or a concealer.
[9] The cosmetic composition according to [7], wherein the cosmetic composition is a sunscreen.

Advantageous Effects of Invention

According to the composite particle of the present invention, a resin particle as a core, gives a soft and smooth feel, and amorphous calcium phosphate and calcium carbonate coated on its surface can effectively absorb sebum, particularly free fatty acid. By blending the composite particle of the present invention into a cosmetic composition, the resulting cosmetic composition can give a smooth feeling upon application and effectively suppress make-up smudges. Also, according to the preferred aspects of the present invention, because unsaturated free fatty acid can be selectively adsorbed, a toning effect on the skin texture can also be anticipated.

Brief Description of Drawings

[Fig. 1] Fig. 1 is a powder X-ray diffraction chart of amorphous calcium phosphate.
[Fig. 2] Fig. 2 is a powder X-ray diffraction chart of crystalline calcium phosphate.
[Fig. 3] Fig. 3 is a scanning electron microscopic photograph of the composite particle obtained in the Production Example 1.
[Fig. 4] Fig. 4 is a scanning electron microscopic photograph of the composite particle obtained in the Production Example 2.

Description of Embodiments

Hereinbelow, the composite particle of the present invention and a method for producing the same as well as a cosmetic composition containing the composite particle will be explained in detail.

1. Composite particle

The composite particle of the present invention is a core-shell-type composite particle comprising a resin particle as a core particle provided on the surface thereof with a coating layer comprising amorphous calcium phosphate and calcium carbonate to form an outer shell.
As used herein, the term "amorphous calcium phosphate" refers to tricalcium phosphate containing water of crystallization represented by a general formula "$Ca_3(PO_4)_2 \cdot nH_2O$", whose crystal structure is amorphous.

The amorphous crystal structure of calcium phosphate can be confirmed by a broader powder X-ray diffractometry pattern than that of crystalline calcium phosphate, which is attributable to high content of water of crystallization in amorphous calcium phosphate (see Fig. 1 and 2).

Also, identification of $Ca_3(PO_4)_2{\cdot}nH_2O$ can be confirmed by pattern fitting with the JCPDS card No. 18-0303.

Within a range of $2\theta$ = 31 ° to 35°, there are three X-ray diffraction peaks in the X-ray diffraction pattern of $Ca_3(PO_4)_2{\cdot}nH_2O$ (JCPDS card No. 18-0303), while there are four X-ray diffraction peaks in the X-ray diffraction pattern of crystalline hydroxyapatite (JCPDS card No. 09-0432); therefore, amorphous calcium phosphate can be distinguished from crystalline apatite by X-ray diffraction measurement.

Having a stronger cohesive force than crystalline calcium phosphate in an aqueous medium, amorphous calcium phosphate aggregates so as to wrap up a resin particle serving as a core particle, thereby forming a strong coating layer after dehydration and drying. Also, compared to crystalline hydroxyapatite and tribasic calcium phosphate, amorphous calcium phosphate generally has a larger specific surface area, thereby exhibiting excellent adsorption performance on the sebum components. Thus, a preventive effect on make-up smudges by adsorption of sebum can be anticipated by inclusion of amorphous calcium phosphate into the coating layer formed on the surface of the resin particle.

Also, as long as the effect of the present invention is not drastically deteriorated, some of the calcium of the aforementioned amorphous calcium phosphate may contain an element such as barium, strontium, zinc, magnesium, sodium, potassium, iron, aluminum, and titanium in the form of a solid solution, or may be ion-exchanged or ion-substituted, and some of the "$PO_4$" may be substituted by one species of atomic group such as "$VO_4$", "$SiO_4$", and "$CO_4$".

Amorphous calcium phosphate used in the present invention can be produced by a publicly known method. Preferred examples include a method described in the formation of a coating layer in the Production Example 1.

As used herein, the term "calcium carbonate" is used to encompass both heavy calcium carbonate obtained by pulverizing a natural product such as limestone and precipitated calcium carbonate obtained by a chemical reaction between a calcium source and a carbon source. According to one embodiment of the present invention, the use of precipitated calcium carbonate is preferred because the particle diameter and the form can be controlled.

Although no particular limitation is imposed on the particle diameter of the calcium carbonate used in the present invention, it is preferably smaller than the composite particle, and is preferably 0.01 μm or more, more preferably 0.05 μm or more, and even more preferably 0.1 μm or more. Also, the particle diameter of the calcium carbonate is preferably 5 μm or less, more preferably 1 μm or less, and even more preferably 0.5 μm or less.

Also, although no particular limitation is imposed on the form of the calcium carbonate used in the present invention, examples thereof include a cube and a sphere.

The calcium carbonate used in the present invention can be produced by a publicly known method. For example, cubic calcium carbonate can be produced by the method described in Japanese Patent Laid-Open No. 2-243515. Also, spherical calcium carbonate can be produced by the method described in Japanese Patent Laid-Open No. 2006-63062.

Further, commercially available calcium carbonate may also be used. For example, various forms of calcium carbonate commercially supplied for cosmetic use by Newlime Co., Ltd. may be preferably used.

Amorphous calcium phosphate and calcium carbonate may each be compounded with one kind or two or more kinds of metallic oxide to form a coating layer.

Although no particular limitation is imposed on the metallic oxide, examples thereof include titanium oxide, zinc oxide, and cerium oxide.

As used herein, the term "core-shell-type" is used to encompass not only a core particle that is completely coated on its surface with an outer shell as a coating layer, but also a core particle that is partially coated on its surface with a coating layer. According to one embodiment of the present invention, a core particle preferably has such a configuration that it is coated with a first layer of amorphous calcium phosphate, which is externally coated with amorphous calcium phosphate and calcium carbonate.

The core particle used in the present invention is a spherical resin particle, which is formed by polymerization of a vinyl monomer so that the average particle diameter is in a range of 5 to 50 μm.

This resin particle contains any one of the following components:

a phosphorous acid diester represented by the following general formula (1):

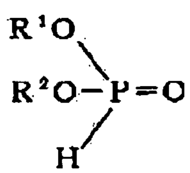 · · · (1)

and a partially esterified phosphoric acid represented by the following general formula (2) or (3):

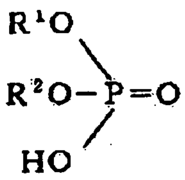 ... (2) 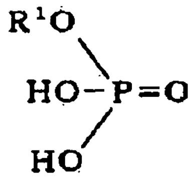 ... (3)

wherein, $R^1$ and $R^2$ in the general formulae (1) to (3) are each independently a monovalent group formed by removal of one hydrogen atom from an acyclic saturated or unsaturated hydrocarbon having 1 to 14 carbon atoms, or a group represented by the following formula (4).

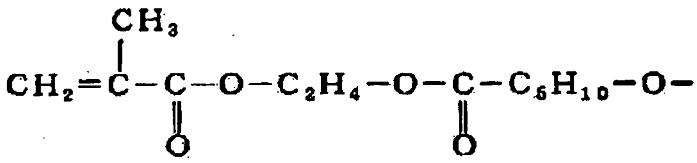 ... (4)

Examples of the aforementioned vinyl monomer include a styrene monomer such as styrene and α-methyl styrene, an acrylic acid ester monomer such as methyl acrylate and butyl acrylate, and a methacrylic acid ester monomer such as methyl methacrylate and butyl methacrylate.

These vinyl monomers can be used for forming a resin particle singly or as a mixture thereof. Further, a monomer other than those described above that can be copolymerized with the above-exemplified monomers can also be used for forming a resin particle.

In order to form a resin particle using the aforementioned monomers, a previously publicly known method such as suspension polymerization and emulsion polymerization can be employed. Among those methods, as a method for forming a resin particle so that sphere having the average particle diameter is in a range of 5 to 50 μm, it is preferable to adopt suspension polymerization in an aqueous medium.

A polymerization initiator can be employed to accelerate polymerization of a vinyl monomer in the above polymerization. As the polymerization initiator, a polymerization initiator soluble in a vinyl monomer that has been conventionally used for suspension polymerization of a vinyl monomer can be adopted.

Examples of such a polymerization initiator include peroxide such as benzoyl peroxide and dicumyl peroxide and an azo compound such as azobisisobutyronitrile.

Also, the average particle diameter of the resin particle can be measured using a precision particle size distribution measuring device such as "Coulter Multisizer II" manufactured by Beckman Coulter Inc. in accordance with "Reference MANUAL FOR THE COULTER MULTISIZER (1987) published by Coulter Electronics Limited" by performing calibration using a 50 μm aperture.

Also, any one of the phosphorous acid ester and the partially esterified phosphoric acid represented by the aforementioned general formulae (1) to (3) can be contained in a resin particle by suspension polymerization.

It is to be noted that phosphorous acid is a substance represented by the following structural formula (5).

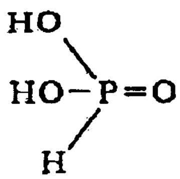 ... (5)

That is, it is a substance having two hydroxy groups that may form an ester bond by a dehydration condensation reaction, etc.

A phosphorous acid ester is a compound represented by the aforementioned general formula (1), which is an esterified phosphorous acid resulting from reactions of all of its two hydroxy groups with alcohols, etc.

That is, the phosphorous acid ester refers exclusively to a fully esterified phosphorous acid, and it does not include a partially esterified phosphorous acid.

Also, $R^1$ and $R^2$ in the general formula (1) represent a monovalent group formed by removal of one hydrogen atom from an acyclic saturated or unsaturated hydrocarbon having 1 to 14 carbon atoms, or a group represented by the aforementioned formula (4).

The $R^1$ and $R^2$ may be the same or different from each other.

Examples of $R^1$ and $R^2$ include a branched or unbranched alkyl group, alkenyl group, and alkynyl group.

Also, preferred examples of the phosphorous acid ester include dibutyl hydrogen phosphite.

Also, the partially esterified phosphoric acid used in the present invention is a partially esterified phosphoric acid represented by "$H_3PO_4$."

It is to be noted that phosphoric acid is a substance represented by the following structural formula (6).

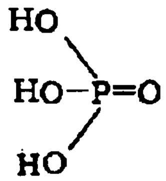 · · · (6)

That is, it is a substance having three hydroxy groups that may form an ester bond by a dehydration condensation reaction, etc.

The partially esterified phosphoric acid is a compound represented by the aforementioned general formula (2) or (3), which is an esterified phosphoric acid resulting from reactions of only one or two of its three hydroxy groups with alcohols, etc.

Here, $R^1$ and $R^2$ are the same as the aforementioned general formula (1), and a fully esterified phosphoric acid is excluded from the compound represented by (2) or (3).

Preferred examples of the partially esterified phosphoric acid include caprolactone EO modified phosphoric acid dimethacrylate, monoisodecyl phosphate, 2-ethylhexyl acid phosphate, and isodecyl acid phosphate.

Here, as caprolactone EO modified phosphoric acid dimethacrylate, a mixture of the compounds represented by the following structural formula (7) in which (n = 1, a = 1, and b = 2) and (n = 1, a = 2, and b = 1), which is a product commercially supplied by Nippon Kayaku Co., Ltd. under the trade name "PM-21", can be used.

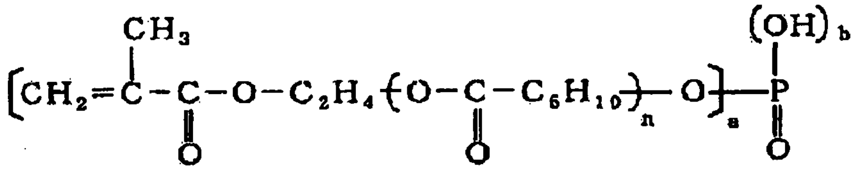 · · · (7)

The aforementioned phosphorous acid ester or partially esterified phosphoric acid (hereinbelow, these substances may also be collectively referred to as "esters") acts as a dispersant of a vinyl monomer in suspension polymerization in an aqueous medium, and upon completion of polymerization, it is in the state of being incorporated in a particle.

Regarding the aforementioned esters used for formation of a resin particle, any only one kind may be used singly or a combination of a plurality thereof may be used. Also, a mixture of one or more kinds selected from the phosphorous acid esters and one or more kinds selected from the partially esterified phosphoric acids can also be used.

Along with these esters, a powder of an inorganic compound can be used as a dispersant in suspension polymerization.

Particularly, it is preferable to use a magnesium pyrophosphate powder.

By carrying out suspension polymerization in an aqueous medium containing these esters and a magnesium pyrophosphate powder, spherical resin particles of a uniform particle diameter can be easily obtained, and so a resin particle useful for the production of a composite particle suitable for a cosmetic can be obtained.

By suspension polymerization using these esters and a magnesium pyrophosphate powder, a polymer (resin particle) can be obtained while suppressing coalescence of vinyl monomers.

Hence, polymerization can be completed in such a state that is close to the initial state of dispersion of the vinyl monomer in an aqueous medium. Also, the size of the resin particle can be adjusted to some degree by modulating the dispersion state of the vinyl monomer in the initial stage of polymerization through adjustment of the proportion of the vinyl polymer, the amount of a dispersant used, the stirring speed in the aqueous medium, etc.

Normally, within the range of 1 to 100 μm, resin particles of uniform size can be obtained by adjustment as described above.

Also, the magnesium pyrophosphate powder is used preferably as a fine powder having an average particle diameter of 0.01 to 10 μm, particularly preferably as a fine powder having an average particle diameter of 0.01 to 1 μm.

Besides the aforementioned magnesium pyrophosphate powder, for example, salts with poor water solubility such as barium carbonate, calcium carbonate, calcium phosphate, and calcium sulfate, and a particle of an inorganic macromolecular substance such as talc, bentonite, silicic acid, diatomaceous earth, and clay can be concomitantly used

as a dispersant.

Similarly to the magnesium pyrophosphate powder, these substances are used preferably as a fine powder having an average particle diameter of 0.01 to 10 μm, particularly preferably as a fine powder having an average particle diameter of 0.01 to 1 μm.

The total amount of esters used is preferably 0.01 to 0.4% by weight relative to the total weight of the vinyl monomer.

Within the aforementioned range, the total amount of esters used is particularly preferably 0.03 to 0.2% by weight.

The reason why the amount of esters used is preferably selected within the aforementioned range is that when the esters are used in an amount of more than 0.4% by weight, in the formation of a monomer droplet by mixing and dispersing an aqueous phase and an oil phase in suspension polymerization, granulation will proceed too far, making it hard to prepare a composite particle of suitable size for cosmetic use.

Also, another reason is that when the amount of esters is less than 0.01% by weight in contrast, there is a risk of formation of a coarse resin particle due to a reduced inhibitory effect on coalescence of vinyl monomer droplets dispersed in the process of polymerization.

Further, the esters also serve both as a component for improving the affinity between a resin particle and amorphous calcium phosphate and as a component as an origin of deposition in the deposition of amorphous calcium phosphate on the surface of a resin particle using a slurry containing a calcium phosphate component.

In view of the foregoing, when the amount of the esters is less than 0.01% by weight, there are risks of disruption of homogeneity in the coating layer of amorphous calcium phosphate and induction of dissociation of the coating layer from the resin particle surface.

Also, the amount of the fine powder of an inorganic compound added is preferably 0.1 to 20% by weight relative to the total weight of the vinyl monomer. Within the aforementioned range, the amount of the fine powder of an inorganic compound added is particularly preferably 0.5 to 10% by weight.

The reason why the amount of the fine powder of an inorganic compound used is preferably selected within the aforementioned range is that when the fine powder of an inorganic compound is used in an amount of more than 20% by weight, the viscosity of a suspension polymerization solution will be too high, making suspension polymerization difficult.

Meanwhile, another reason is that when the amount of the fine powder of an inorganic compound is less than 0.1% by weight, there is a risk of formation of a coarse resin particle due to a reduced inhibitory effect on coalescence of vinyl monomer droplets.

Further, although a detailed description is omitted here, it is also possible to include a component other than those exemplified above, for example a coloring component, in the formation of a resin particle and a coating layer. It is also possible to further externally form a colored layer, etc. over the coating layer formed by amorphous calcium phosphate.

In the composite particle of the present invention, the weight ratio of calcium phosphate is preferably 5 parts by weight or more, more preferably 10 parts by weight or more relative to 100 parts by weight of the resin particle. Also, the weight ratio of calcium phosphate is preferably 30 parts by weight or less, more preferably 25 parts by weight or less.

Also, in the composite particle of the present invention, the weight ratio of calcium carbonate is preferably 3 parts by weight or more, more preferably 5 parts by weight or more relative to 100 parts by weight of the resin particle. Also, the weight ratio of calcium carbonate is preferably 20 parts by weight or less, more preferably 10 parts by weight or less.

Also, the average particle diameter of the composite particle of the present invention is preferably 1 μm or more, more preferably 2 μm or more, and even more preferably 5 μm or more. Also, the average particle diameter of the composite particle of the present invention is preferably 100 μm or less, more preferably 50 μm or less, and even more preferably 20 μm or less.

Also, the average particle diameter of the composite particle can be obtained by a similar method used for the resin particle, for example, by using a precision particle size distribution measuring device such as "Coulter Multisizer II" manufactured by Beckman Coulter Inc. in accordance with "Reference MANUAL FOR THE COULTER MULTISIZER (1987) published by Coulter Electronics Limited" by performing calibration using a 50 μm aperture.

Next, a method for producing a resin particle and a method for producing a core-shell-type composite particle using the resin particle obtained by the method as a core particle will be explained.

Method for producing a resin particle

As a method for producing a resin particle according to the present embodiment, suspension polymerization using the components exemplified above is preferably carried out.

Although no particular limitation is imposed on the order of mixing of each component, etc., the mixing is preferably performed in the following order. That is,

<Step 1-1>

First of all, a powder of an inorganic compound such as a magnesium pyrophosphate powder and a surfactant, etc., if necessary, are dissolved in water in advance.

<Step 1-2>

Separately, a liquid mixture in which the esters, a polymerization initiator, and a vinyl monomer are mixed is produced.

<Step2>

Subsequently, the aqueous dispersion of an inorganic compound powder prepared by Step 1-1 and the liquid mixture prepared by Step 1-2 are mixed and stirred to produce a suspension of a liquid droplet of a vinyl monomer dispersed in an aqueous medium.

It should be noted that in Step 2, the resin particle of the desired size can be easily obtained by keeping the stirring speed constant once the size of the particle has reached the desired size through modulation of stirring speed of a mixer or a homomixer while observing the size of the particle (droplet) of the vinyl polymer, and thereafter, continuing stirring at the same stirring speed.

In order to produce a resin particle by causing a polymerization reaction in the resulting suspension, an autoclave is preferably used as a reaction container.

As the autoclave, one with a mixer and having a jacket enabling heating or cooling is preferred.

Also, it is preferable to raise the temperature to 50 to 100°C in the initial stage of polymerization, and thereafter, maintain the temperature within the above range, and continue stirring without stopping during the period of polymerization.

Thereafter, under the condition in which the heating temperature and stirring are maintained as above, a vinyl monomer is polymerized with time, and normally, polymerization can be completed in several hours to give a polymer (resin particle). During this time, coalescence between the dispersed vinyl monomer particles can be inhibited, thereby giving resin particles of the desired uniform particle size.

Whether or not coalescence has occurred can be easily determined by taking out some of the polymer particles thus obtained, observing them under a microscope, and checking to see if a large number of irregularly shaped particles are present.

Also, at this time of polymerization of a vinyl monomer, a resin particle is formed while incorporating the aforementioned esters.

Describing this in detail, a relatively large number of the esters are present near the interface between the liquid droplet formed by a vinyl monomer in an aqueous medium and the aqueous medium (i.e., the surface of the particle) before polymerization, and as the polymerization reaction of the vinyl monomer proceeds, a part of the ester, particularly the hydrocarbon moiety or a moiety corresponding to the aforementioned formula (4) is plunged into the particle, whereby the ester is incorporated into the resin particle, notably in a state such that a part thereof is reacted with the vinyl monomer.

These esters have a high affinity to calcium phosphate, and the site where the esters are present is likely to serve as the origin of deposition of amorphous calcium phosphate.

Moreover, the abundant presence of dispersed esters on the surface of the resin particle promotes homogeneous deposition of amorphous calcium phosphate, and as calcium carbonate attaches to the amorphous calcium phosphate thus deposited, a homogeneous coating layer is formed.

Also, as the coating layer is homogenized, a composite particle is also easily formed into a similar spherical shape to the resin particle, whereby a spherical composite particle having a homogeneous coating layer that is suitable for a cosmetic is easily formed.

Method for producing a composite particle

Subsequently, a method for producing a composite particle using this resin particle will be explained.

No particular limitation is imposed on the method for forming a coating layer of amorphous calcium phosphate and calcium carbonate on the surface of this resin particle; however, in order to form a homogeneous coating layer, it is important to form a coating layer by producing an aqueous suspension of the aforementioned resin particle and the calcium phosphate component, depositing amorphous calcium phosphate on the surface of the aforementioned resin particle, and allowing calcium carbonate to attach to the resulting amorphous calcium phosphate.

Examples of the method for producing such a composite particle as described above include a method of performing the steps such as ones described below. That is,

<Step A>

Producing an alkali solution by dispersing a calcium salt such as calcium hydroxide in water.

<Step B>

Producing a dispersion solution of resin particle by dispersing the resin particle produced by the aforementioned production method in the alkali solution produced by Step A.

<Step C>

Forming a coating layer by adding phosphoric acid to the dispersion solution of resin particle produced by Step B so that it composes a calcium phosphate component with calcium of the calcium hydroxide added as above, and depositing calcium phosphate on the surface of the resin particle in an aqueous suspension containing the resulting calcium phosphate component.

At this time, because there is a risk of formation of calcium hydrogen phosphate when the pH of the solution containing the calcium phosphate component becomes acidic, particularly, below pH 5.0, it is preferable to maintain the pH of the aforementioned aqueous suspension at 5.0 or above in the formation of a coating layer of calcium phosphate.

Particularly, it is preferable to adjust the amount of phosphoric acid added so that the pH value is anywhere between 6.5 and 10.5.

Also, because the temperature of the aqueous suspension is elevated due to the reaction heat generated by the addition of phosphoric acid, for example, it is preferable to form a coating layer while cooling the aqueous suspension so as to maintain the temperature at 50°C or below.

By performing cooling as above, the cumbersome work can be omitted, namely the step of adding phosphoric acid does not have to be divided in multiple steps and there is no need to wait for the suspension to naturally cool down after each step of adding phosphoric acid, thereby making the production method of a composite particle more efficient.

Also, in the deposition of amorphous calcium phosphate in the above Step C, the esters contained in the resin particle are likely to serve as the origin of deposition of amorphous calcium phosphate, and because this origin (esters), which is advantageous for the deposition of amorphous calcium phosphate, is present abundantly on the surface of the resin particle, deflection in the deposition speed of amorphous calcium phosphate on the surface of the resin particle is less likely to occur, whereby a homogeneous coating layer is formed.

Moreover, the adhesion between the coating layer and the resin particle is enhanced, thereby preventing amorphous calcium phosphate from detaching from the surface of the composite particle.

<Step D>

To a slurry containing a resin particle, on the surface of which amorphous calcium phosphate is deposited by Step C (a mixed slurry of the resin particle, on the surface of which amorphous calcium phosphate is deposited, and a fine particle of amorphous calcium phosphate), a slurry of calcium carbonate is added. The resulting slurry is mixed by stirring to give a slurry containing a mixture of the resin particle, on the surface of which amorphous calcium phosphate and calcium carbonate are deposited, a fine particle of amorphous calcium phosphate, and a fine particle of calcium carbonate.

It is to be noted that the amount of amorphous calcium phosphate supported on the resin particle can be mainly adjusted by the ratio between the resin particle and the calcium phosphate component in Step C.

The amount of calcium carbonate supported on the resin particle can be adjusted by the ratio between the resin particle and the calcium carbonate component in Step D.

At this time, when the contents of the calcium phosphate component and the calcium carbonate component are too small, a sufficient thickness of the coating layer cannot be secured, leading to a fear that amorphous calcium phosphate and calcium carbonate may fail to fully exert the light-scattering property and the organic substance-adsorption property in the composite particle to be obtained. Meanwhile, when excess amounts of the calcium phosphate component and the calcium carbonate component are included, there is a fear that a large number of single particles of each of amorphous calcium phosphate and calcium carbonate may be formed.

From the foregoing perspective, although depending on the size of the resin particle, etc., for example, in the case of a resin particle having an average particle diameter of 1 to 50 $\mu$m, the calcium phosphate component is preferable contained in the aqueous suspension at a ratio of 5 parts by weight or more, and also 10 parts by weight or more relative to 100 parts by weight of the resin particle, while the calcium phosphate component is preferably contained in the aqueous suspension at a ratio of 30 parts by weight or less, and also 25 parts by weight or less.

Also, the calcium carbonate component is preferably contained in the aqueous suspension at a ratio of 3 parts

by weight or more, and also 5 parts by weight or more relative to 100 parts by weight of the resin particle having the aforementioned average particle diameter, while the calcium carbonate component is preferably contained in the aqueous suspension at a ratio of 20 parts by weight or less, and also 10 parts by weight or less.

<Step E>

After forming a coating layer of certain thickness on the surface, the aqueous suspension can be subjected to dehydration drying to provide a core-shell-type composite particle in a powdery state.

Dehydration drying strengthens the cohesive force of amorphous calcium phosphate and calcium carbonate forming a coating layer, by which the coating layer is more strongly supported to the surface, preventing itself from falling off.

It is to be noted that because the composite particle before drying is in such a state that the coating layer is swollen with water, it is softer than the coating layer after drying.

Therefore, for example, in such a case that a dehydration cake is produced by filtering the aforementioned aqueous suspension using a dehydration device, a composite particle powder obtained by drying a composite particle having a somewhat hydrated coating layer in a somewhat soft state while stirring, etc. ends up with a less bumpy surface than a composite particle powder obtained by crushing the dried dehydration cake thus obtained.

For example, in the step of depositing amorphous calcium phosphate on the surface of the resin particle, although amorphous calcium phosphate is relatively homogeneously deposited by the action of the esters, there is still a possibility of formation of some degree of bumpiness on the surface of the composite particle.

Also, besides depositing on the surface of a resin particle, some of the amorphous calcium phosphate singly deposits as a particle of finer size than the resin particle in a similar manner to calcium carbonate. Thus, if this aqueous suspension is filtered, a state may develop in which single particles of such amorphous calcium phosphate and calcium carbonate are attached to the surface of the composite particle.

In that case, when the dehydration cake that has been separated by filtration is left to dry as is or after merely crushing it to some degree, bumpiness on the surface and protrusions due to attachment of the amorphous calcium phosphate particles and calcium carbonate particles will remain on the dried composite particle.

Meanwhile, by subjecting a composite particle immediately after separation by filtration by a dehydration device that still has a soft coating layer to the step of drying with stirring, the composite particles are allowed to collide and rub against each other before the coating layer is hardened as drying proceeds. By doing so, the bumps formed on the surface of the composite particle right after the step of separation by filtration are averaged out and the fine particles of amorphous calcium phosphate and calcium carbonate attached to the surface are incorporated into the coating layer, etc., whereby the surface condition can turn into flat.

The composite particle obtained as above has a flat surface, and thus is particularly suitable for a cosmetic. For example, when it is used as a component of a make-up cosmetic, it can reduce a frictional feeling when the make-up cosmetic is spread over the skin, thereby helping a user to attain a smooth feeling.

Also, although depending on the size of the composite particle and the thickness of the coating layer, etc., normally, the water content in the state of dehydration cake is approximately 15 to 70% by weight.

Given the above, in order to obtain a dry powder having a flat surface as mentioned above, it is preferable to perform drying while continuously stirring the entire contents until at least the aforementioned water content is reduced to 5% by weight or less, and it is more preferable to continue stirring until the water content is reduced to 2% by weight or less.

At this time, there is a fear that if drying is performed too rapidly, the dehydration cake may be dried before the surface of the composite particle is sufficiently flattened, whereas if drying is performed too gently, operation time may be prolonged.

In view of the foregoing, the time required to bring the water content of a dehydration cake containing 15 to 70% by weight of water to 5% by weight or less is preferably adjusted to be approximately 5 to 20 hours.

The step of drying with stirring as described above can be performed by, for example, using a stirring-type dryer, etc.

As described above, coating of the surface with amorphous calcium phosphate and calcium carbonate can be easily done by using a resin particle containing the asters.

Moreover, a composite particle made in such a way that amorphous calcium phosphate and calcium carbonate are prevented from detaching from its surface can be obtained by using the aforementioned resin particle.

The composite particle of the present invention obtained as above cannot only impart a soft feeling upon application by using a resin particle as a core particle, but also selectively adsorb sebum, particularly, free fatty acid, by concomitantly using amorphous calcium phosphate and calcium carbonate as a coating layer of the resin particle, thereby effectively inhibiting make-up smudges. Also, according to the preferred embodiments of the present invention, a toning effect on the skin texture can also be anticipated since the composite particle selectively adsorbs unsaturated free fatty

acid. Thus, the composite particle of the present invention is suitable as a component to be blended in a cosmetic.

2. Cosmetic composition

The cosmetic composition of the present invention comprises: a core-shell-type composite particle comprising a resin particle as a core particle having, formed on the surface thereof, a coating layer comprising amorphous calcium phosphate and calcium carbonate; and a cosmetically acceptable component

As the composite particle used in the cosmetic composition of the present invention, ones described in the aforementioned "1. Composite particle" can be used.

Although the content of the composite particle in the cosmetic composition of the present invention varies depending on the purpose and usage, normally, it is preferably 0.1% by weight or more, more preferably 0.5% by weight or more, and even more preferably 1 % by weight or more relative to the total weight of the cosmetic composition. Also, the content of the composite particle is preferably 40% by weight or less, more preferably 35% by weight or less, and even more preferably 30% by weight or less.

For example, when the cosmetic composition of the present invention is used as a powder foundation, the content of the composite particle is preferably 0.1% by weight or more, more preferably 0.5% by weight or more, and even more preferably 1% by weight or more relative to the total weight of the cosmetic composition. Also, the content of the composite particle is preferably 40% by weight or less, more preferably 35% by weight or less, and even more preferably 30% by weight or less.

Also, for example, when the cosmetic composition of the present invention is used as a liquid foundation (such as W/Si type or O/W type), the content of the composite particle is preferably 0.1% by weight or more, more preferably 0.5% by weight or more, and even more preferably 1 % by weight or more relative to the total weight of the cosmetic composition. Also, the content of the composite particle is preferably 35% by weight or less, more preferably 25% by weight or less, and even more preferably 20% by weight or less.

Also, for example, when the cosmetic composition of the present invention is used as an eye shadow, the content of the composite particle is preferably 0.1% by weight or more, more preferably 0.5% by weight or more, and even more preferably 1% by weight or more relative to the total weight of the cosmetic composition. Also, the content of the composite particle is preferably 40% by weight or less, more preferably 30% by weight or less, and even more preferably 20% by weight or less.

Also, for example, when the cosmetic composition of the present invention is used as a blush, the content of the composite particle is preferably 0.1% by weight or more, more preferably 0.5% by weight or more, and even more preferably 1% by weight or more relative to the total weight of the cosmetic composition. Also, the content of the composite particle is preferably 30% by weight or less, more preferably 20% by weight or less, and even more preferably 10% by weight or less.

Also, for example, when the cosmetic composition of the present invention is used as a loose powder, the content of the composite particle is preferably 0.1% by weight or more, more preferably 0.5% by weight or more, and even more preferably 1 % by weight or more relative to the total weight of the cosmetic composition. Also, the content of the composite particle is preferably 40% by weight or less, more preferably 35% by weight or less, and even more preferably 25% by weight or less.

Also, for example, when the cosmetic composition of the present invention is used as a make-up base, the content of the composite particle is preferably 0.1% by weight or more, more preferably 0.5% by weight or more, and even more preferably 1% by weight or more relative to the total weight of the cosmetic composition. Also, the content of the composite particle is preferably 30% by weight or less, more preferably 20% by weight or less, and even more preferably 10% by weight or less.

Also, for example, when the cosmetic composition of the present invention is used as a concealer, the content of the composite particle is preferably 0.1% by weight or more, more preferably 0.5% by weight or more, and even more preferably 1% by weight or more relative to the total weight of the cosmetic composition. Also, the content of the composite particle is preferably 30% by weight or less, more preferably 20% by weight or less, and even more preferably 10% by weight or less.

Also, for example, when the cosmetic composition of the present invention is used as a sunscreen, the content of the composite particle is preferably 0.1% by weight or more, more preferably 0.5% by weight or more, and even more preferably 1% by weight or more relative to the total weight of the cosmetic composition. Also, the content of the composite particle is preferably 30% by weight or less, more preferably 20% by weight or less, and even more preferably 10% by weight or less.

When the amount of the composite particle blended is too small, it might be difficult to obtain the desired sebum absorption effect, whereas when the amount of the composite particle blended is too much, the sebum absorption effect can be obtained but it gives an excessively dry feeling, resulting in reduced adhesion to skin.

As long as the purpose and effect of the present invention are not impaired, the cosmetic composition of the

present invention can further contain a cosmetically acceptable component in addition to the composite particle of the present invention.

Examples of the cosmetically acceptable components include powder components other than the composite particles of the present invention, a liquid oil, a solid fat, a wax, a hydrocarbon, a higher fatty acid, a higher alcohol (preferably an alcohol having 6 or more carbon atoms, and more preferably an alcohol having 10 or more carbon atoms), a Synthetic ester oil, a silicone oil, a surfactant, a co-surfactant, a moisturizing agent, a film forming agent, a thickener, a gelling agent, an inorganic mineral, a metal sequestering agent, a lower alcohol, a polyhydric alcohol, a monosaccharide, an oligosaccharide, an amino acid, a plant extract, an organic amine, a polymer emulsion, an antioxidant, an antioxidant aid, a skin nutrient, a vitamin, a blood flow promoter, an antibacterial agent, an anti-inflammatory agent, a cell (skin) activating agent, a keratolytic agent, a refrigerant, a water-soluble polymer, a skin whitening agent, a UV absorber, an anti-fading agent, an antiseptic agent, a skin softener, an antiaging agent, an anti-pollution agent, a keratolytic agent, a pH adjustor, a buffer, a perfume and water.

Any of the above-described components may be suitably selected and blended depending on a desired formulation and a product form. The blending amount of the components is not particularly limited as long as it can be used without departing from the purpose of the present invention. The blending amount is suitably selected depending on a formulation, a product form, etc.

Examples of the powder components include inorganic powders, such as talc, kaolin, mica, sericite, white mica, gold mica, a synthetic mica, red mica, black mica, vermiculite, magnesium carbonate, calcium carbonate, aluminum silicate, barium silicate, calcium silicate, magnesium silicate, strontium silicate, a metal tungstate, silica, zeolite, barium sulfate, magnesium sulfate, calcined calcium sulfate (calcined gypsum), calcium phosphate, fluorine apatite, hydroxyapatite, ceramic powder, metallic soap (e.g. zinc myristate, calcium palmitate, aluminum stearate, magnesium stearate and boron nitride); organic powders, such as polyamide resin powder (nylon powder), polyethylene powder, polymethyl methacrylate powder, polystyrene powder, styrene/acrylic acid copolymer resin powder, benzoguanamine resin powder, polytetrafluoroethylene powder and cellulose powder; metal powder pigments, such as aluminum powder and copper powder; organic pigments, such as a zirconium-, barium-, or aluminum-lakes; and natural colors, such as chlorophyll and β-carotene. Note that the powder components may be hydrophobized.

Examples of the liquid oil include avocado oil, camellia oil, turtle oil, macadamia nut oil, corn oil, mink oil, olive oil, rapeseed oil, yolk oil, sesame oil, persic oil, wheat germ oil, camellia kissi oil, castor oil, linseed oil, safflower oil, cotton seed oil, perilla oil, soybean oil, peanut oil, tea seed oil, Torreya seed oil, rice bran oil, Chinese tung oil, Japanese tung oil, jojoba oil, germ oil, and triglycerin.

Examples of the solid fat include cacao butter, coconut oil, horse tallow, hardened coconut oil, palm oil, palm kernel oil, Japan tallow kernel oil, hardened oil, Japan tallow, and hardened castor oil.

Examples of the wax include bees wax, candelilla wax, cotton wax, carnauba wax, bayberry wax, Chinese insect wax, montan wax, bran wax, lanolin, kapok wax, lanolin acetate, liquid lanolin, sugar cane wax, lanolin fatty acid isopropyl ester, hexyl laurate, reduction lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of the hydrocarbon oil include liquid paraffin, ozokerite, squalane, pristane, paraffin, ceresin, squalene, vaseline, microcrystalline wax, and hydrogenated polydecene.

Examples of the higher fatty acid include lauric acid, myristic acid, palmitic acid, stearic acid, behenic acid, oleic acid, undecylenie acid, tall oil acid, isostearic acid, linoleic acid, linolenic acid, eicosapentaenoic acid (EPA), and docosahexaenoic acid (DHA).

Examples of the higher alcohol include linear alcohols, such as lauryl alcohol, cetyl alcohol, stearyl alcohol, behenyl alcohol, myristyl alcohol, oleyl alcohol, and cetostearyl alcohol; branched alcohols, such as monostearyl glycerin ether (batyl alcohol), 2-decyltetradecanol, lanolin alcohol, cholesterol, phytosterol, hexyldodecanol, isostearyl alcohol, and octyldodecanol.

Examples of the synthetic ester oil include tripropylene glycol dineopentanoate, isononyl isononanoate, isotridecyl isononanoate, isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkylglycol monoisostearate, neopentyl glycol dicaprylate, diisostearyl malate, glycerin di-2-heptylundecanoate, trimethylolpropane tri-2-ethylhexanoate, trimethylolpropane triisostearate, pentaerythritol tetra-2-ethylhexanoate, glycerin tri-2-ethylhexanoate, glycerin trioctanoate, glycerin triisopalmitate, trimethylolpropane triisostearate, cetyl 2-ethylhexanoate, 2-ethylhexyl palmitate, glycerin trimyristate, glyceride tri-2-heptylundecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, bis-behenyl/isostearyl/phytosteryl dimer dilinoleyl dimer dilinoleate, phytosteryl/behenyl/octyldodecyl/isostearyl lauroyl glutamate, and

tri(caprylic acid/capric acid) glyceryl.

Examples of the silicone oil include a chain polysiloxane, such as dimethicone, methyl trimethicone, methylphenylpolysiloxane and diphenylpolysiloxane; a cyclic polysiloxane, such as octamethyl cyclotetrasiloxane, decamethyl cyclopentasiloxane and dodecamethyl cyclohexasiloxane; a silicone resin forming a 3D net structure; a silicone rubber; a silicone elastomer; various modified polysiloxanes, such as amino-modified polysiloxane, polyether-modified polysiloxane, alkyl-modified polysiloxane and fluorine-modified polysiloxane.

Examples of the silicone resin include acrylates/polytrimethylsiloxymethacrylate copolymers commercially available from DOW CORNING Corporation, under the trade name of, for example, DC FA 4001 and DC FA 4002, and the like. Other examples of the silicone resin include trimethylsiloxysilicate/dimethiconol crosspolymers commercially available from DOW CORNING Corporation, under the trade name of, for example, DC 7-4411.

Examples of silicone elastomers include non-emulsifying organopolysiloxane elastomers or emulsifying organosiloxane elastomers. Examples of the non-emulsifying organopolysiloxane elastomers include dimethicone/vinyl dimethicone crosspolymers, lauryl dimethicone/vinyl dimethicone crosspolymers, and the like.

The dimethicone/vinyl dimethicone crosspolymers include products commercially available from DOW CORNING Corporation, Midland, Michigan, under the trade name of, for example, DC 9040 and DC 9045; products commercially available from MOMENTIVE Performance Materials Inc. under the trade name of SFE 839 and the Velvasil series products; products commercially available from SHIN-ETSU Chemical Co., Ltd. under the trade name of, for example, KSG-15, KSG-16, and KSG-18 ([dimethicone/phenyl vinyl dimethicone crosspolymer]); and Gransil (TM) series products from GRANT INDUSTRIES, Inc.

The lauryl dimethicone/vinyl dimethicone crosspolymers include products commercially available from SHIN-ETSU Chemical Co., Ltd. under the trade name of, for example, KSG-31, KSG-32, KSG-41, KSG-42, KSG-43, and KSG-44.

Examples of the emulsifying organosiloxane elastomers include polyalkoxylated silicone elastomers, polyglycerolated silicone elastomers, or the like.

The polyalkoxylated silicone elastomers include products commercially available from DOW CORNING Corporation under the trade name of, for example, DC9010 and DC9011, products commercially available from SHIN-ETSU Chemical Co., Ltd. under the trade name of, for example, KSG-20, KSG-21, KSG-30, KSG-31, KSG-32, KSG-33, KSG-210, KSG-310, KSG-320, KSG-330, KSG-340, and X-226146.

The polyglycerolated silicone elastomers include products commercially available from SHIN-ETSU Chemical Co., Ltd. under the trade name of, for example, KSG-710, KSG-810, KSG-820, KSG-830, KSG-840, KSG-31, KSG-32, KSG-41, KSG-42, KSG-43, and KSG-44. In addition, examples of silicone elastomers into which 2 types of branches, i.e., a silicone chain and an alkyl chain have been introduced include products commercially available from SHIN-ETSU Chemical Co., Ltd. under the trade name of, for example, KSG-042Z, KSG-045Z, KSG-320Z, KSG-350Z, KSG-820Z, and KSG-850Z.

Silicone elastomers comprising a polyalkyl ether group as pendant or cross linked may also included as components in the cosmetic composition of the present invention. Particularly suitable silicone elastomers comprising a polyalkyl ether group include compounds with the International Nomenclature of Cosmetic Ingredients (INCI) name: bis-vinyldimethicone/bis-isobutyl PPG-20 crosspolymer, bis-vinyldimethicone/PPG-20 crosspolymer, dimethicone/bis-isobutyl PPG-20 crosspolymer, dimethicone/PPG-20 crosspolymer, and dimethicone/bis-sec butyl PPG-20 crosspolymer. Such cross-linked elastomers are available from Dow Coming Corporation under the experimental names of SOEB-1, SOEB-2, SOEB-3 and SOEB-4, etc., and under the proposed commercial name of DC EL-8052 IH Si Organic Elastomer Blend, etc. The elastomer particles are supplied pre-swollen in the respective solvents, isododecane (for SOEB-1 and SOEB-2), isohexadecane (for SOEB-3), and isodecyl neopentanoate (for SOEB-4).

Examples of the surfactant include a lipophilic nonionic surfactant and a hydrophilic nonionic surfactant.

Examples of the lipophilic nonionic surfactant include a sorbitan fatty acid ester, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate and diglycerol sorbitan tetra-2-ethylhexylate; a glycerin polyglycerin fatty acid, such as glycerin mono-cotton seed oil fatty acid, glycerin monoerucate, glycerin sesquioleate, glycerin monostearate, glycerin $\alpha,\alpha'$-oleate pyroglutamate, and glycerin monostearate malate; a propylene glycol fatty acid ester such as monostearate propylene glycol; a hardened castor oil derivative; and a glycerin alkyl ether.

Examples of the hydrophilic nonionic surfactant include a POE-sorbitan fatty acid ester, such as POE-sorbitan monooleate, POE-sorbitan monostearate, POE-sorbitan monooleate and POE-sorbitan tetraoleate; a POE sorbitol fatty acid ester, such as POE-sorbitol monolaurate, POE-sorbitol monooleate, POE-sorbitol pentaoleate and POE-sorbitol monostearate; a POE-glycerin fatty acid ester, such as POE-glycerin monostearate, POE-glycerin monoisostearate and POE-glycerin triisostearate; a POE-fatty acid ester, such as POE-monooleate, POE-distearate, POE-monodioleate and ethylene glycol distearate; a POE-alkyl ether, such as POE-lauryl ether, POE-oleyl ether, POE-stearyl ether, POE-behenyl ether, POE-2-octyldodecyl ether and POE-cholestanol ether; a Pluronic type surfactant (e.g., Pluronic); a POE-POP-alkyl ether, such as POE-POP-cetyl ether, POE-POP-2-decyltetradecyl ether, POE-POP-monobutyl ether, POE-POP-hydrogenated lanolin and POE-POP-glycerin ether.

Examples of the co-surfactants include higher alcohols. Among them, linear alcohols such as lauryl alcohol, cetyl alcohol, stearyl alcohol, behenyl alcohol, myristyl alcohol, oleyl alcohol, cetostearyl alcohol, and the like, are preferable. Cetyl alcohol is particularly preferable.

Examples of the metal sequestering agent include 1-hydroxyethane-1,1-diphosphonic acid; 1-hydroxyethane-1,1-diphosphonic acid tetrasodium salt; disodium edetate; trisodium edetate; tetrasodium edetate; sodium citrate; sodium polyphosphate; sodium metaphosphate; gluconic acid; phosphoric acid; citric acid; ascorbic acid; succinic acid; edetic acid; and trisodium ethylenediamine hydroxyethyl triacetate.

Examples of the lower alcohol include ethanol, propanol, isopropanol, isobutyl alcohol, and t-butyl alcohol.

Examples of the polyhydric alcohol include a dihydric alcohol, such as ethylene glycol, propylene glycol, pentylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol and octylene glycol; a trihydric alcohol, such as glycerin and trimethylolpropane; a tetrahydric alcohol such as pentaerythritol (e.g., 1,2,6-hexanetriol); a pentahydric alcohol such as xylitol; a hexahydric alcohol, such as sorbitol and mannitol; a polyhydric alcohol polymer, such as diethylene glycol, dipropylene glycol, triethylene glycol, polypropylene glycol and tetraethylene glycol; a dihydric alcohol alkyl ether, such as ethylene glycol monomethyl ether and ethylene glycol monoethyl ether; a dihydric alcohol alkyl ether, such as diethylene glycol monomethyl ether, diethylene glycol monoethyl ether and diethylene glycol monobutyl ether; a dihydric alcohol ether ester, such as ethylene glycol monomethyl ether acetate and ethylene glycol monoethyl ether acetate; a glycerin monoalkyl ether, such as chimyl alcohol, selachyl alcohol and batyl alcohol; and a sugar alcohol, such as sorbitol, maltitol, maltotriose, mannitol, sucrose, erythritol, glucose, fructose, starch sugar, maltose, xylitose, and a reduced alcohol of a starch sugar.

Examples of the monosaccharide include a triose, such as D-glyceryl aldehyde and dihydroxyacetone; a tetrose, such as D-erythrose, D-erythrulose, D-threose and erythritol; a pentose, such as L-arabinose, D-xylose, L-lyxose, D-arabinose, D-ribose, D-ribulose, D-xylulose and L-xylulose; a hexose, such as D-glucose, D-talose, D-psicose, D-galactose, D-fructose, L-galactose, L-mannose and D-tagatose; a heptose, such as aldoheptose and heprose; an octose such as octurose; a deoxy sugar, such as 2-deoxy-D-ribose, 6-deoxy-L-galactose and 6-deoxy-L-mannose; an amino sugar, such as D-glucosamine, D-galactosamine, sialic acid, amino uronic acid and muramic acid; a uronic acid, such as D-glucuronic acid, D-mannuronic acid, L-guluronic acid, D-galacturonic acid and L-iduronic acid.

Examples of the oligosaccharide include sucrose, lactose, maltose, trehalose, cellobiose, gentiobiose, umbilicin, raffinose, gentianose, maltotriose, melezitose, planteose, unbelliferose, stachyose, and verbascose.

Examples of the amino acid include a neutral amino acid, such as threonine and cysteine; and a basic amino acid such as hydroxylysine. Further, as an amino acid derivative, for example, sodium acyl sarcosinate (sodium lauroyl sarcosinate), acyl glutamate, sodium acyl β-alanine, glutathione, and pyrrolidone carboxylic acid may be exemplified.

Examples of the organic amine include monoethanolamine, diethanolamine, triethanolamine, morpholine, triisopropanolamine, 2-amino-2-methyl-1,3-propanediol, and 2-amino-2-methyl-1-propanol.

Examples of the polymer emulsion include an acrylic resin emulsion, a poly(ethyl acrylate) emulsion, an acrylic resin solution, a poly(alkyl acrylate) emulsion, a poly(vinyl acetate) resin emulsion, and a natural rubber latex.

Examples of the vitamin include vitamins A, $B_1$, $B_2$, $B_6$, C and E and derivatives thereof, pantothenic acid and derivatives thereof and biotin.

Examples of the antioxidants include ascorbic acid and its derivatives such as ascorbyl palmitate, ascorbyl tetraisopalmitate, ascorbyl glucoside, magnesium ascorbyl phosphate, sodium ascorbyl phosphate and ascorbyl sorbate; tocopherol and its derivatives, such as tocopherol acetate, tocopherol sorbate, and other esters of tocopherol; dibutyl hydroxytoluene (BHT) and butyl hydroxyanisole (BHA), gallic acid ester, phosphoric acid; citric acid; maleic acid; malonic acid; succinic acid; fumaric acid; cephalin; a hexametaphosphate; phytic acid; ethylenediaminetetraacetic acid; and plant extracts, for instance from Chondrus crispus, Rhodiola, Thermus thermophilus, mate leaves, oak wood, kayu rapet bark, sakura leaves and ylang ylang leaves.

Examples of the moisturizing agent include polyethylene glycol; propylene glycol; dipropylene glycol; glycerin; 1,3-butylene glycol; xylitol; sorbitol; maltitol; mucopolysaccharides such as chondroitin sulfuric acid; hyaluronic acid; mucoitinsulfuric acid; caronic acid; atelo-collagen; cholesteryl-12-hydroxystearate; bile salt; a main component ofNMF (natural moisturizing factor), such as a pyrrolidone carboxylic acid salt and a lactic acid salt; amino acids such as urea, cysteine and serine; short-chain soluble collagen; a diglycerin (EO) PO addition product; homo- or copolymers of 2-methacryloyloxyethylphosphorylcholine commercially available from NOF CORPORATION under the trade name of, for example, Lipidure HM and Lipidure PBM; panthenol; allantoin; PEG/PPG/Polybutylene Glycol-8/5/3 Glycerin commercially available from NOF CORPORATION under the trade name of Wilbride S 753; Trimethylglycine commercially available from Asahi KASEI Chemicals Corporation under the trade name of AMINOCOAT; and various plant extracts such as Castanea sativa extracts, hydrolyzed hazelnut proteins, Polianthes tuberosa polysaccharides, Argania spinosa kernel oil, and an extract of pearl containing conchiolin commercially available from Maruzen Pharmaceuticals Co. LTD. under the trade name of Pearl Extract ®.

Examples of the skin softener include glyceryl polymethacrylate, methyl gluceth-20 and the like.

Examples of the antiaging agent include acyl amino acids (specifically, products commercially available from SEDERMA under the trade name of Maxilip, Matrixyl 3000 or Biopeptide CL, or product commercially available from SEPPIC under the trade name of Sepilift); Pisum sativum extracts; hydrolyzed soy proteins; methylsilanol mannuronate; hydrolyzed cucurbita pepo seedcake; Scenedesmus extract; and the like.

Examples of the anti-pollution agents include Moringa pterygosperma seed extracts (specifically, product commercially available from LSN under the trade name of Purisoft); Shea butter extract (specifically, products commercially available from SILAB under the trade name of Detoxyl, a blend of ivy extract, phytic acid and sunflower seed extract (for example, product commercially available from SEDERMA under the trade name of OSMOPUR)), and the like.

Examples of the keratolytic agents include α-hydroxy acids (specifically, glycolic, lactic, citric, malic, mandelic or tartaric acid), β-hydroxy acids (specifically, salicylic acid), esters thereof (specifically, $C_{12-13}$ alkyl lactate), and plant extracts containing these hydroxy acids (specifically, Hibiscus sabdriffa extracts), and the like.

Examples of the water-soluble polymer include dextrin, methylcellulose, ethylcellulose, sodium carboxymethylcellulose, hydroxyethyl cellulose, hydroxypropyl cellulose, hydroxypropylmethylcellulose stearoyl ester, PVA, PVM, PVP, locust bean gum, guar gum, tara gum, tamarind gum, glucomannan, xylan, mannan and agar.

Examples of the anti-inflammatory agents include bisabolol, allantoin, tranexamic acid, zinc oxide, sulfur oxide and its derivatives, chondroitin sulfate, and glycyrrhizic acid and its derivatives (for example, glycyrihizinates).

The cosmetic composition of the present invention may contain at least one whitening agent to block the synthesis of structural proteins such as the melanocyte-specific protein Pmell7 involved in the mechanism of melanogenesis (stage I). Example of such a whitening agent may include the ferulic acid-containing cytovector (water, glycol, lecithin, ferulic acid, hydroxyethylcellulose) commercially available from BASF under the trade name of Cytovector®.

Furthermore, if necessary, the cosmetic composition of the present invention may contain at least one peptide as described in International Publication WO2009/010356 pamphlet.

Furthermore, if necessary, the cosmetic composition of the present invention may include a whitening agent having an inhibition effect on melanin synthesis and/or an inhibition effect on nanophthalmia-related transcription factor (MITF) expression and/or an anti-tyrosinase activity and/or an inhibition effect on endothelin-1 synthesis. Examples of such a whitening agent include Glycyrrhiza glabra extract commercially available from Maruzen Pharmaceuticals Co. LTD. under the trade name of Licorice extract ®.

Furthermore, if necessary, the cosmetic composition of the present invention may include whitening agents having an antioxidant effect as well, such as vitamin C compounds, which include ascorbate salts, ascorbyl esters of fatty acids or of sorbic acid, and other ascorbic acid derivatives. Specific examples include ascorbyl phosphates (magnesium ascorbyl phosphate, sodium ascorbyl phosphate, and the like), and saccharide esters of ascorbic acid (ascorbyl-2-glucoside, 2-O-α-D-glucopyranosyl L-ascorbate, 6-O-β-D-galactopyranosyl L-ascorbate, and the like). Active agents of this type are commercially available from DKSH under the trade name of Ascorbyl glucoside ®.

Furthermore, if necessary, the cosmetic composition of the present invention may include other whitening agents. Examples of the other whitening agents may include pigmentation inhibiting agents such as plant extracts (e.g;, Narcissus tazetta extracts), cetyl tranexamate (Nikko Chemicals Co., Ltd; trade name: NIKKOL TXC), arbutin, kojic acid, ellagic acid, cysteine, 4-thioresorcin, resorcinol or rucinol or their derivatives, glycyrrhizic acid, hydroquinone-β-glucoside, and the like.

Furthermore, if necessary, the cosmetic composition of the present invention may also include organic and/or inorganic sunscreens.

Examples of the organic sunscreens include dibenzoylmethane derivatives such as butyl methoxydibenzoylmethane (product commercially available from HOFFMANN LA ROCHE Ltd. under the trade name of Parsol 1789); cinnamic acid derivatives such as octyl methoxycinnamate (product commercially available from HOFFMANN LA ROCHE Ltd. under the trade name of Parsol MCX), salicylates, para-aminobenzoic acids; β,β'-diphenylacrylate derivatives; benzophenone derivatives; benzylidenecamphor derivatives such as terephthalylidene dicamphor sulphonic acid; phenylbenzimidazole derivatives; triazine derivatives; phenylbenzotriazole derivatives; anthranilic acid derivatives, and the like, all of which may be coated or encapsulated.

Examples of the inorganic sunscreens may include pigments or nanopigments formed from coated or uncoated metal oxides. Examples of the nanopigments include titanium oxide, iron oxide, zinc oxide, zirconium oxide or cerium oxide, which are all well known as UV photoprotective agents.

Examples of the antiseptic agent include p-hydroxybenzoate ester (e.g., methylparaben and propylparaben) and phenoxyethanol.

In addition, as additives to be used in the cosmetic composition of the present invention, those mentioned in International Cosmetic Ingredient Dictionary and Handbook, 13th Edition, 2010, published by the Personal Care Products Council, can be used.

The formulation of the cosmetic composition of the present invention is arbitrarily selectable, and a preferred formulation may be adopted according to the product form. For example, a formulation such as a solution type, an

emulsion type, a powder dispersion type, a water-oil bilayer type, a water-oil-powder trilayer type, a gel type, and an oil type can be adopted.

The cosmetic composition of the present invention is obtainable by combining the composite particle of the present invention with any cosmetically acceptable component according to the desired formulation and production form, and mixing these components by a method commonly employed for the preparation of a cosmetic composition. The cosmetic composition of the present invention may be shaped as needed.

The cosmetic composition of the present invention is used in the form of, for example, a make-up cosmetic (such as a foundation, an eye shadow, a blush, a mascara, a lip make-up product, a body make-up product, and a nail product) and a skin care cosmetic (such as an emulsion, a cream, and a sunscreen). Among them, the cosmetic composition of the present invention is preferably used as a skin make-up cosmetic composition, particularly a foundation (such as a powder foundation and a liquid foundation), an eye shadow, a blush, a loose powder, and a concealer, and the like.

In the preparation of these cosmetic compositions, the composite particle of the present invention may be additionally blended into a conventional formulation or may be blended in substitution for a conventionally blended sebum-absorbing powder. For the formulation of a cosmetic composition, see Shin Keshohingaku (literal translation: New Cosmetology), edited by Takeo Mitsui, published by Nanzando Co. Ltd. (Second edition, published on January 18, 2001), and the like.

## Examples

Hereinbelow, the present invention will be specifically explained with reference to Examples and Comparative Examples; however, the present invention is not limited to these Examples.

### 1. Preparation of a composite particle

The composite particle of the present invention was prepared by the method described in Production Example 1. Subsequently, the hydroxyapatite-coated methyl methacrylate crosspolymer, which is the composite particle described in Japanese Patent Laid-Open No. 2010-241785, was produced by the method described in Production Example 2.

### (1) Production Example 1 (the composite particle of the invention of the present application)

<Production of a resin particle>

Into a stainless steel beaker with a capacity of 5 L, a dispersion solution obtained by dispersing 60 g of a magnesium pyrophosphate powder as a dispersant in 3000 g of water was placed.

In a separate container, 50 g of ethylene glycol dimethacrylate and 0.9 g of 2,2'-azobis(2,4-dimethylvaleronitrile) were added to 950 g of methyl methacrylate, to which caprolactone EO modified phosphoric acid dimethacrylate (manufactured by Nippon Kayaku Co., Ltd., trade name "PM-21") was further added as the esters at a ratio of 500 ppm/monomer to produce a monomer solution.

The monomer solution thus produced was placed in the aforementioned stainless steel beaker and the resulting mixture was dispersed by the "benchtop TK homomixer" (revolution, 6000 rpm) manufactured by Tokushu Kika Kogyo Co., Ltd. Subsequently, the resulting dispersion solution was placed in a jacket-type autoclave (capacity of 5 L) with a mixer, and suspension polymerization was carried out for six hours while stirring and maintaining the temperature in the autoclave at 50°C.

Subsequently, the temperature was raised to 105°C and stirring was continued for two hours to produce a slurry containing a resin particle.

Subsequently, the slurry was cooled, to which 6 N hydrochloric acid was added until the pH of the slurry was 1 or less, and magnesium pyrophosphate was dissolved, followed by filtration and washing.

The average particle diameter of the resin particle thus obtained was 8.5 μm.

<Formation of a coating layer>

In ion-exchanged water, 1000 g of the resin particles thus produced were dispersed, and the total volume was made up to 3.5 L. To this, 80 g of calcium hydroxide was added, followed by thorough stirring to produce a resin particle dispersion solution. The pH of the resin particle dispersion solution thus obtained was 13.0.

The above resin particle dispersion solution was cooled to a temperature of 20°C. or less, to which orthophosphoric acid diluted to a concentration of 10% was gradually added until the pH reached 10.5 to produce an aqueous suspension containing a calcium phosphate component.

It should be noted that the temperature of the resin particle dispersion solution was adjusted so as not to exceed 40°C while adding orthophosphoric acid, and the number of stirring revolutions was appropriately adjusted according to the changes in the viscosity of the resin particle dispersion solution.
After completion of dropwise addition of orthophosphoric acid, stirring was continued for two hours.
Subsequently, the calcium carbonate slurry prepared in advance was added, followed by further stirring continued for two hours, whereby amorphous calcium phosphate and calcium carbonate were deposited on the surface of the resin particle. The resulting aqueous suspension was then filtered and dried to obtain a composite particle.
The average particle diameter of the composite particle thus obtained was 8.7 μm. The scanning electron microscopic photograph of the composite particle thus obtained is shown in Fig. 3.

<Preparation of a calcium carbonate slurry>

Into a PE beaker with a capacity of 1000 ml, 500 ml of ion-exchanged water and 50 g of calcium carbonate (cubic calcium carbonate NL-RC01 (Newlime Co., Ltd.), an average particle diameter of 0.2 μm) were added, and the resulting mixture was dispersed by "benchtop TK homomixer" manufactured by Tokushu Kika Kogyo Co., Ltd. at 6000 rpm for five minutes to prepare an aqueous slurry of calcium carbonate,

(2) Production Example 2: Production of a hydroxyapatite-coated methyl methacrylate crosspolymer

<Production of a resin particle>

Following a similar method to Production Example 1, a resin particle was produced.

<Formation of a coating layer>

In ion-exchanged water, 1000 g of the resin particle thus produced was dispersed, and the total volume was made up to 3.5 L. To this, 80 g of calcium hydroxide was added, followed by thorough stirring to produce a resin particle dispersion solution. Also, the pH of the resin particle dispersion solution thus obtained was 1.3.0.
The above resin particle dispersion solution was cooled to the temperature of 20°C or less, to which orthophosphoric acid diluted to a concentration of 10% was gradually added until the pH reached 10.5 to produce an aqueous suspension containing a calcium phosphate component.
It should be noted that the temperature of the resin particle dispersion solution was adjusted so as not to exceed 40°C while adding orthophosphoric acid, and the number of stirring revolutions was appropriately adjusted according to the changes in the viscosity of the resin particle dispersion solution.
After completion of dropwise addition of orthophosphoric acid, stirring was continued for two hours to deposit amorphous calcium phosphate on the surface of the resin particle. The resulting aqueous suspension was then filtered and dried to obtain a composite particle.
Also, the dried composite particle was crushed by a commercially available mixer and filtered through a 200 mesh sieve to obtain a hydroxyapatite-coated methyl methacrylate crosspolymer particle. The scanning electron microscopic photograph of the composite particle thus obtained is shown in Fig. 4.

Method for measuring the average particle diameter (the volume average particle diameter) of the resin particle and the composite particle

The volume average particle diameter (arithmetic mean diameter in the volume-based particle size distribution) of the resin particle and the composite particle is measured by Coulter Multisizer II (a measuring device manufactured by Beckman Coulter Inc.). At this time, the measurement is performed in accordance with "Reference MANUAL FOR THE COULTER MULTISIZER (1987) published by Coulter Electronics Limited" by performing calibration using a 50 μm aperture.
Specifically, 0.1 g of the resin particle (or 0.1 g of the composite particle) was preliminary dispersed in 10 ml of a 0.1 % by weight nonionic surfactant using a touch mixer ("TOUCHMIXER MT-31" manufactured by Yamato Scientific Co., Ltd.) and an ultrasonic cleaner ("ULTRASONIC CLEANER VS-150" manufactured by VELVO-CLEAR) to obtain a dispersion solution. Subsequently, into a beaker filled with ISOTON® II (Beckman Coulter Inc.: an electrolytic solution for measurement) attached to the body of Coulter Multisizer II, the aforementioned dispersion solution is added dropwise using a pipette while gently stirring so that the densitometer on the front side of the body of Coulter Multisizer II indicates around 10%. Subsequently, the following entries were inputted into the body of Coulter Multisizer II; the aperture size (diameter), 50 μm. Current (aperture current), 800 μA, Gain, 4, and Polarity (polarity of the inner electrode), +, and the measurement is performed manually (manual mode). During the measurement, the inside of the beaker was stirred

gently so as not to generate air bubbles in the beaker, and the measurement is completed when 100 thousand particles are measured. The volume average particle diameter (arithmetic mean diameter in the volume-based particle size distribution) is an average value of 100 thousand particle diameter.

2. Evaluation of the selective adsorptivity of the composite particle for free fatty acid

Using the composite particle obtained in Production Example 1, the selective adsorptivity of the composite particle of the present invention for free fatty acid was evaluated in the following manner.

To 10 g of the composite particle obtained in Production Example 1,100 g of an oil sample was added, followed by stirring. Subsequently; the composite particle was removed by a glass filter.

Subsequently, the residual amount of free fatty acid in the oil was calculated by measuring the acid value by the following measurement method.

Selective adsorption of free fatty acid to the composite particle and the amount of free fatty acid thus adsorbed, can be confirmed from the degree of reduction in the acid value of oil after adsorption treatment using the composite particle relative to the acid value of oil before adsorption treatment.

For comparison, the selective adsorptivity of the composite particle for free fatty acid was similarly evaluated also in the cases in which methyl methacrylate crosspolymer, hydroxyapatite, the hydroxyapatite-coated methyl methacrylate crosspolymer obtained in Production Example 2, and calcium carbonate were used in substitution for the composite particle.

It should be noted that the "acid value" as used herein refers to the number of mg of potassium hydroxide necessary for neutralizing free fatty acid contained in 1 g of oil.

Method for measuring the acid value

From the filtered oil sample, 10 g was weighted out, to which 100 ml of an ethanol/ether mixed solution was added, in which the oil sample was dissolved. To this, a few drops of a phenolphthalein reagent was added as an indicator, and titration was performed using a 0.01 mol/L solution of potassium hydroxide in ethanol until the solution of oil sample exhibited a pink color that persisted for 30 seconds.

The acid value was calculated from the amount of the 0.01 mol/L solution of potassium hydroxide in ethanol necessary for titration by the following formula.

$$\text{Acid value} = a \times F \times 5.611 / \text{amount of the oil sample (g)}$$

a: Amount of the 0.01 mol/L solution of potassium hydroxide in ethanol (ml) F: Titer of the 0.01 mol/L solution of potassium hydroxide in ethanol (0.086)

The ethanol/ether mixed solution used for measurement was prepared by mixing 99.5 vol% ethanol and diethyl ether at a ratio of 1 : 2 (volume ratio).

Also, the 0.01 mol/L solution of potassium hydroxide in ethanol was prepared by dissolving 7.0 g of potassium hydroxide in 5 ml of water, adding 95 vol% ethanol to this solution to make it up to 1,000 ml (0.1 mol/L solution of potassium hydroxide in ethanol), and further, diluting the resulting solution 10-fold with 95 vol% ethanol (0.01 mol/L solution of potassium hydroxide in ethanol).

The results are shown in Table 1.

**Table 1**

| | | Oil-1 (corn salad oil) | | Oil-2 (mixed Oil)*1 | |
|---|---|---|---|---|---|
| | | Acid value | Residual amount of free fatty acid (% by weight) | Acid value | Residual amount of free fatty acid (% by weight) |
| Oil before adsorption treatment | | 0.19 | 100 | 0.22 | 100 |
| Oil after adsorption treatment | Methyl methacrylate crosspolymer*2 | 0.19 | 100 | 0.22 | 100 |
| | Hydroxyapatite*3 | 0.11 | 58 | 0.11 | 50 |
| | Hydroxyapatite-coated methyl methacrylate crosspolymer | 0.10 | 53 | 0.11 | 50 |
| | Calcium carbonate*4 | 0.09 | 47 | 0.10 | 45 |
| | Composite particle of the present invention | 0.072 | 38 | 0.072 | 33 |

*1 Composition of Oil-2 (Mixed oil):

(Caprylic capric acid) triglyceryl 56.2% by weight

Octyldodecyl myristate 31.2% by weight

Squalane 12.5% by weight

Oleic acid 0.1% by weight

*2 TECHPOLYMER MBX-8C (SEKISUI PLASTICS CO., Ltd.)

*3 plate-like HAP: (Taihei Chemical Industrial Co. Ltd.)

*4 calcium carbonate (Newlime Co., Ltd.)

As shown in Table 1, the methyl methacrylate crosspolymer adsorbs oil but does not selectively adsorb fatty acid. Consequently, there is no change in the residual amount of free fatty acid in oil after adsorption treatment

Meanwhile, the hydroxyapatite powder and the calcium carbonate powder were each found to reduce the residual amount of free fatty acid in oil after adsorption treatment to approximately 50% by weight.

The composite particle of the present invention was found to reduce the residual amount of free fatty acid in oil after adsorption treatment to approximately 30 to 40% by weight.

From the above results, it can be assumed that the composite particle of the present invention has a remarkably higher selective adsorption effect on free fatty acid than conventional sebum-absorbing powders.

Also, in the test conducted using Oil-2 (mixed oil), the composite particle of the present invention was confirmed to be capable of selectively adsorbing oleic acid, which is unsaturated free fatty acid.

3. Preparation of a cosmetic composition

Using the composite particle obtained in Production Example 1, various kinds of cosmetic compositions having the following compositions were prepared.

Example 1 and Comparative Examples 1 and 2 (powder foundations)

<Production method>

The components (16) to (20) were mixed and added to the components (1) to (15) that had been mixed in advance. Subsequently, the mixture thus obtained was pulverized by a hammer mill (manufactured by Dalton Co., Ltd.),

and a certain amount was pressed by a powder press (manufactured by Sanshin Seiki Co., Ltd.) to obtain a powder foundation.

| Formulation Example of a powder foundation | | Example 1 | Comparative Example 1 | Comparative Example 2. |
|---|---|---|---|---|
| (1) | Hydrophobized titanium oxide | 13.0 | 13.0 | 13.0 |
| (2) | Hydrophobized iron oxide | 2.0 | 2.0 | 2.0 |
| (3) | Hydrophobized talc | 30.0 | 30.0 | 30.0 |
| (4) | Hydrophobized mica | 3.0 | 3.0 | 3.0 |
| (5) | Nylon powder | 2.0 | 2.0 | 2.0 |
| (6) | (Vinyl dimethicone/methicone silsesquioxane) crosspolymer | 15.0 | 15.0 | 15.0 |
| (7) | (HDI/trimethylol hexyllactone) crosspolymer | 5.0 | 5.0 | 5.0 |
| (8) | Hydroxyapatite-calcium carbonate-coated methyl methacrylate crosspolymer | 6.0 | 0.0 | 0.0 |
| (9) | Hydroxyapatite*5 | 0.0 | 6.0 | 0.0 |
| (10) | Methyl methacrylate crosspolymer*6 | 0.0 | 0.0 | 6.0 |
| (11) | Chlorphenesin | 0.4 | 0.4 | 0.4 |
| (12) | Sodium dehydroacetate | 0.4 | 0.4 | 0.4 |
| (13) | Potassium sorbate | 0.5 | 0.5 | 0.5 |
| (14) | Magnesium myristate | 1.0 | 1.0 | 1.0 |
| (15) | Silica | 5.0 | 5.0 | 5.0 |
| (16) | Octyl methoxycinnamate | 7.5 | 7.5 | 7.5 |
| (17) | Trioctanoin | 2.1 | 2.1 | 2.1 |
| (18) | Squalane | 4.0 | 4.0 | 4.0 |
| (19) | Dimethicone | 3.0 | 3.0 | 3.0 |
| (20) | Tocopherol acetate | 0.1 | 0.1 | 0.1 |

*5 plate-like HAP (Taihei Chemical Industrial Co. Ltd.)
*6 TECHPOLYMER MBX-8C (SEKISUI PLASTICS CO., Ltd.)

Example 2 and Comparative Examples 3 and 4(loose powders)

<Production method>

The components (14) to (17) were mixed and added to the components (1) to (13) that had been mixed in advance. Subsequently, the components (1) to (17) were pulverized by a hammer mill (manufactured by Dalton Co., Ltd.) to obtain a loose powder.

| Formulation Example of a loose powder | | Example 2 | Comparative Example 3 | Comparative Example 4 |
|---|---|---|---|---|
| (1) | Hydrophobized titanium oxide | 15.0 | 15.0 | 15.0 |
| (2) | Hydrophobized iron oxide | 4.0 | 4.0 | 4.0 |
| (3) | Hydrophobized talc | 8.0 | 8.0 | 8.0 |
| (4) | Hydrophobized sericite | 20.0 | 20.0 | 20.0 |
| (5) | Synthetic gold mica | 20.0 | 20.0 | 20.0 |
| (6) | Cellulose | 5.0 | 5.0 | 5.0 |
| (7) | Boron nitride | 12,0 | 12.0 | 12.0 |
| (8) | Hydroxyapatite-calcium carbonate-coated methyl methacrylate crosspolymer | 4.0 | 0.0 | 0.0 |
| (9) | Hydroxyapatite*5 | 0.0 | 4.0 | 0.0 |
| (10) | Methyl methacrylate crosspolymer*6 | 0.0 | 0.0 | 4.0 |

(continued)

| Formulation Example of a loose powder | | Example 2 | Comparative Example 3 | Comparative Example 4 |
|---|---|---|---|---|
| (11) | Lauroyllysine | 6.0 | 6.0 | 6.0 |
| (12) | Chlorphenesin | 0.3 | 0.3 | 0.3 |
| (13) | Sodium dehydroacetate | 0.3 | 0.3 | 0.3 |
| (14) | Squalane | 3.0 | 3.0 | 3.0 |
| (15) | Dimethicone | 1.3 | 1.3 | 1.3 |
| (16) | Vaseline | 1.0 | 1.0 | 1.0 |
| (17) | Tocopherol acetate | 0.1 | 0.1 | 0.1 |

*5 plate-like HAP (Taihei Chemical Industrial Co. Ltd.)
*6 TECHPOLYMER MBX-8C (SEKISUI PLASTICS CO., Ltd.)

Example 3 and Comparative Examples 5 and 6 (W/Si type foundations)

<Production method>

The components (1) to (10) were uniformly mixed and dispersed to prepare an oil phase. The components (11) to (15) were mixed and dissolved at 70°C. The resulting product was cooled, to which the component (16) was added to prepare an aqueous phase. The aqueous phase was added little by little to the oil phase thus obtained while thoroughly stirring. At last, the components (17) to (19) were added while stirring, and the resulting mixture was mixed to uniformity. The resulting liquid mixture was subjected to degassing to obtain a W/Si type foundation.

| Formulation Example of a W/Si type foundation | | Example 3 | Comparative Example 5 | Comparative Example 6 |
|---|---|---|---|---|
| (1) | PEG-10 Dimethicone | 2.0 | 2.0 | 2.0 |
| (2) | (Dimethicone/(PEG-10/15))crosspolymer | 1.0 | 1.0 | 1.0 |
| (3) | Methyl trimethicone | 26.3 | 26.3 | 26.3 |
| (4) | Tocopherol acetate | 0.1 | 0.1 | 0.1 |
| (5) | Octyl methoxycinnamate | 7.5 | 7.5 | 7.5 |
| (6) | Polymethylsilsesquioxane | 4.0 | 4.0 | 4.0 |
| (7) | Dimethicone | 3.0 | 3.0 | 3.0 |
| (8) | Hydrophobized titanium oxide | 11.0 | 11.0 | 11.0 |
| (9) | Hydrophobized iron oxide | 2.0 | 2.0 | 2.0 |
| (10) | Hydrophobized talc | 3.0 | 3.0 | 3.0 |
| (11) | Ion-exchanged water | 30.0 | 30.0 | 30.0 |
| (12) | 1,3-Butylene glycol | 3.0 | 3.0 | 3.0 |
| (13) | Phenoxyethanol | 0.4 | 0.4 | 0.4 |
| (14) | Magnesium sulfate | 0.5 | 0.5 | 0.5 |
| (15) | Methylparaben | 0.2 | 0.2 | 0.2 |
| (16) | Ethanol | 3.0 | 3.0 | 3.0 |
| (17) | Hydroxyapatite-calcium carbonate-coated methyl methacrylate crosspolymer | 3.0 | 0.0 | 0.0 |
| (18) | Hydroxyapatite*6 | 0.0 | 3.0 | 0.0 |
| (19) | Methyl methacrylate crosspolymer*7 | 0.0 | 0.0 | 3.0 |

*6 plate-like HAP (Taihei Chemical Industrial Co. Ltd.)
*7 TECHPOLYMER MBX-8C (SEKISUI PLASTICS CO., Ltd.)

Example 4 and Comparative Examples 7 and 8 (O/W type foundations)

<Production method>

The components (1) to (13) were uniformly mixed and dispersed, and then heated to 80°C to prepare an oil phase. Next, the components (14) to (19) were mixed and dissolved at 80°C to prepare an aqueous phase. The aqueous

phase was added little by little to the oil phase thus obtained while thoroughly stirring. The resulting mixture was stirred to uniformity, and then cooled. Finally, the components (20) to (22) were added to this composition, followed by stirring. The composition thus obtained was subjected to degassing to obtain an O/W type foundation.

| Formulation Example of an O/W type foundation | | Example 4 | Comparative Example 7 | Comparative Example 8 |
|---|---|---|---|---|
| (1) | Squalane | 1.5 | 1.5 | 1.5 |
| (2) | Glyceryl isostearate | 1.0 | 1.0 | 1.0 |
| (3) | Caprylyl glycol | 0.2 | 0.2 | 0.2 |
| (4) | Stearic acid | 1.5 | 1.5 | 1.5 |
| (5) | Caprylyl methicone | 6.0 | 6.0 | 6.0 |
| (6) | Behenyl alcohol | 0.5 | 0.5 | 0.5 |
| (7) | Cetanol | 1.0 | 1.0 | 1.0 |
| (8) | Isoceteth-20 | 1.0 | 1.0 | 1.0 |
| (9) | Hydrophobized titanium oxide | 15.0 | 15.0 | 15.0 |
| (10) | Hydrophobized iron oxide | 2.5 | 2.5 | 2.5 |
| (11) | Hydrophobized talc | 3.0 | 3.0 | 3.0 |
| (12) | Octyl methoxycinnamate | 7.5 | 7.5 | 7.5 |
| (13) | Acrylates/polytrimethylsil oxymethacrylate copolymer | 1.0 | 1.0 | 1.0 |
| (14) | Butylene glycol | 4.5 | 4.5 | 4.5 |
| (15) | Bentonite | 2.0 | 2.0 | 2.0 |
| (16) | Phenoxyethanol | 0.9 | 0.9 | 0.9 |
| (17) | Chlorphenesin | 0.2 | 0.2 | 0.2 |
| (18) | Ion-exchanged water | 47.3 | 47.3 | 47.3 |
| (19) | Triethanolamine | 0.4 | 0.4 | 0.4 |
| (20) | Hydroxyapatite calcium carbonate-coated methyl methacrylate crosspolymer | 3.0 | 0.0 | 0.0 |
| (21) | Hydroxyapatite *5 | 0.0 | 3.0 | 0.0 |
| (22) | Methyl methacrylate crosspolymer *6 | 0.0 | 0.0 | 3.0 |

*5 plate-like HAP (Taihei Chemical Industrial Co. Ltd.)
*6 TECHPOLYMER MBX-8C (SEKISUI PLASTICS CO., Ltd.)

Example 5 and Comparative Examples 9 and 10 (eye shadows)

[Production method]

The components (12) to (13) were mixed and added to the components (1) to (11) that had been mixed in advance. Subsequently, the components (1) to (13) were mixed by a mixer and a certain amount was pressed by a powder press (manufactured by Sanshin Seiki Co., Ltd.) to obtain an eye shadow.

| Formulation Example of an eye shadow | | Example 5 | Comparative Example 9 | Comparative Example 10 |
|---|---|---|---|---|
| (1) | Hydrophobized talc | 7.7 | 7.7 | 7.7 |
| (2) | Hydrophobized mica | 10.0 | 10.0 | 10.0 |
| (3) | Magnesium stearate | 9.0 | 9.0 | 9.0 |
| (4) | Boron nitride | 7.0 | 7.0 | 7.0 |
| (5) | Pearlescent agent | 50.0 | 50.0 | 50.0 |
| (6) | Hydroxyapatite calcium carbonate-coated methyl methacrylate crosspolymer | 4.0 | 0.0 | 0.0 |
| (7) | Hydroxyapatite*5 | 0.0 | 4.0 | 0.0 |
| (8) | Methyl methacrylate crosspolymer*6 | 0.0 | 0.0 | 4.0 |
| (9) | Nylon 12 | 3.0 | 3.0 | 3.0 |

(continued)

| Formulation Example of an eye shadow | | Example 5 | Comparative Example 9 | Comparative Example 10 |
|---|---|---|---|---|
| (10) | Lauroyl lysine | 4.0 | 4.0 | 4.0 |
| (11) | Chlorphenesin | 0.2 | 0.2 | 0.2 |
| (12) | Squalane | 5.0 | 5.0 | 5.0 |
| (13) | Tocopherol acetate | 0.1 | 0.1 | 0.1 |

*5 plate-like HAP (Taihei Chemical Industrial Co. Ltd.)
*6 TECHPOLYMER MBX-8C (SEKISUI PLASTICS CO., Ltd.)

Example 6 and Comparative Examples 11 and 12 (sunscreens)

<Production method>

The components (1) to (11) were uniformly mixed and dispersed to prepare an oil phase. Subsequently, the components (12) to (15) were mixed to prepare an aqueous phase. The aqueous phase was added little by little to the oil phase thus obtained while thoroughly stirring. At last, the components (16) to (18) were added to this composition while stirring, and the resulting mixture was mixed to uniformity. The resulting liquid mixture was subjected to degassing to obtain a sunscreen.

| Formulation Example of a sunscreen | | Example 6 | Comparative Example 11 | Comparative Example 12 |
|---|---|---|---|---|
| (1) | PEG-9 Polydimethylsiloxyethyl dimethicone | 2.0 | 2.0 | 2.0 |
| (2) | Caprylyl methicone | 9.0 | 9.0 | 9.0 |
| (3) | Methyl trimethicone | 18.5 | 18.5 | 18.5 |
| (4) | Neopentyl glycol dicaprate | 4.0 | 4.0 | 4.0 |
| (5) | Isononyl isononanoate | 4.0 | 4.0 | 4.0 |
| (6) | Octyl methoxycinnamate | 7.5 | 7.5 | 7.5 |
| (7) | Caprylyl glycol | 0.5 | 0.5 | 0.5 |
| (8) | (Dimethicone/vinyl Dimethicone)crosspolymer | 4.0 | 4.0 | 4.0 |
| (9) | Polymethylsilaesquioxane | 3.0 | 3.0 | 3.0 |
| (10) | Hydrophobized titanium oxide | 3.0 | 3.0 | 3.0 |
| (11) | Hydrophobized zinc oxide | 22.0 | 22.0 | 22.0 |
| (12) | Ion-exchanged water | 10.0 | 10.0 | 10.0 |
| (13) | Dipropylene glycol | 5.0 | 5.0 | 5.0 |
| (14) | Ethanol | 5.0 | 5.0 | 5.0 |
| (15) | Phenoxyethanol | 0.5 | 0.5 | 0.5 |
| (16) | Hydroxyapatite-calcium carbonate-coated methyl methacrylate crosspolymer | 2.0 | 0.0 | 0.0 |
| (17) | Hydroxyapatite*5 | 0.0 | 2.0 | 0.0 |
| (18) | Methyl methacrylate crosspolymer*6 | 0.0 | 0.0 | 2.0 |

*5 plate-like HAP (Taihei Chemical Industrial Co. Ltd.)
*6 TECHPOLYMER MBX-8C (SEKISUI PLASTICS CO., Ltd.)

4. Evaluation of the cosmetic compositions

The cosmetic compositions obtained in Examples 1 to 6 and Comparative Examples 1 to 12 were evaluated for the feeling upon application and cosmetic durability based on the following evaluation criteria.

Evaluation criteria

<Feeling upon application>

Ten female expert panelists evaluated each kind of the cosmetic compositions for the feeling upon application based on the following evaluation criteria. It is to be noted that the cosmetic compositions that were evaluated as having no coarseness but having good spreadability and smoothness were defined as having a good feeling upon application in the evaluation of the feel upon application conducted by the subjects.

A: Eight or more out of 10 acknowledged a good feeling upon application.
B: Seven out of 10 acknowledged a good feeling upon application.
C: Four to six out of 10 acknowledged a good feeling upon application.
D: Three or less out of 10 acknowledged a good feeling upon application.

<Cosmetic durability>

Ten female expert panelists evaluated each kind of the cosmetic compositions for cosmetic durability after application based on the following evaluation criteria. It is to be noted that the cosmetic compositions that did not produce oily shine due to sebum, wear off, or cause a color change were defined as having good cosmetic durability in the evaluation of the condition 10 hours after application conducted by the subjects.

A: Eight or more out of 10 felt good cosmetic durability.
B: Seven out of 10 felt good cosmetic durability.
C: Four to six out of 10 felt good cosmetic durability.
D: Three or less out of 10 felt good cosmetic durability.

<Skin texture>

Ten female expert panelists evaluated each kind of the cosmetic compositions for skin texture after application based on the following evaluation criteria. It is to be noted that the cosmetic compositions that were evenly spread over the bumpy surface of the skin, etc. to give a finely textured, even finish after application were defined as giving good skin texture.

A: Eight or more out of 10 acknowledged good skin texture.
B: Seven out of 10 acknowledged good skin texture.
C: Four to six out of 10 acknowledged good skin texture.
D: Three or less out of 10 acknowledged good skin texture.

The results are shown in Table 2.

Table 2

| | | Feeling upon application | Cosmetic durability | Skin texture |
|---|---|---|---|---|
| Powder foundation | Example 1 | A | A | A |
| | Comparative Example 1 | D | A | D |
| | Comparative Example 2 | B | D | B |
| Loose powder | Example 2 | A | B | A |
| | Comparative Example 3 | D | B | D |
| | Comparative Example 4 | A | D | B |
| W/Si type foundation | Example 3 | A | A | A |
| | Comparative Example 5 | D | B | C |
| | Comparative Example 6 | A | C | B |

(continued)

| | | Feeling upon application | Cosmetic durability | Skin texture |
|---|---|---|---|---|
| O/W type foundation | Example 4 | A | A | A |
| | Comparative Example 7 | D | B | C |
| | Comparative Example 8 | B | C | B |
| Eye shadow | Example 5 | A | A | A |
| | Comparative Example 9 | D | B | D |
| | Comparative Example 10 | B | D | B |
| Sunscreen | Example 6 | A | A | A |
| | Comparative Example 11 | D | B | C |
| | Comparative Example 12 | B | C | B |

As a result, the products of Examples containing the composite particle of the present invention were clearly smoother, had a better feeling upon application, and exhibited better cosmetic durability owing to high selective adsorption effects for free fatty acid than the products of Comparative Examples. Also, the products of Examples gave good skin texture due to the spherical particle and its surface condition.

The products of Comparative Examples containing hydroxyapatite exhibited poor spreadability and gave a coarse feel, resulting in a poor feeling upon application. Moreover, they gave an unfavorable skin texture. However, they exhibited good cosmetic durability owing to the sebum-adsorbing effect of hydroxyapatite.

Also, the products of Comparative Examples containing a methyl methacrylate crosspolymer spread well, were smooth, and achieved a good feeling upon application; however, due to their lack of adsorption effects, cosmetic durability was poor. However, owing to the spherical particle, the skin texture was not bad.

Industrial Applicability

The composite particle of the present invention is smooth and gives a good feeling upon application, and can selectively adsorb sebum, particularly, free fatty acid. Therefore, it is suitable as a component contained in various kinds of cosmetic composition, particularly a make-up cosmetic such as a foundation, an eye shadow, a concealer, a blush, a loose powder, and a make-up base; and a skin care cosmetic such as a sunscreen.

## Claims

1. A core-shell-type composite particle comprising: a resin particle as a core particle having, formed on the surface thereof, a coating layer comprising amorphous calcium phosphate and calcium carbonate,
   wherein the resin particle contains a polymer of a vinyl monomer and any one of the following components:

   a phosphorous acid diester represented by the following general formula (1):

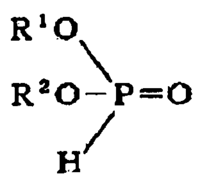

· · · (1)

   and a partially esterified phosphoric acid represented by the following general formula (2) or (3):

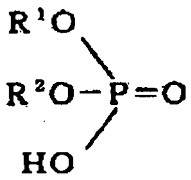 • • • (2) 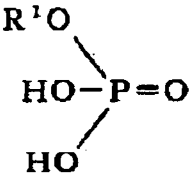 • • • (3)

wherein, $R^1$ and $R^2$ in the general formulae (1) to (3) are each independently a monovalent group formed by removal of one hydrogen atom from an acyclic saturated or unsaturated hydrocarbon having 1 to 14 carbon atoms, or a group represented by the following formula (4):

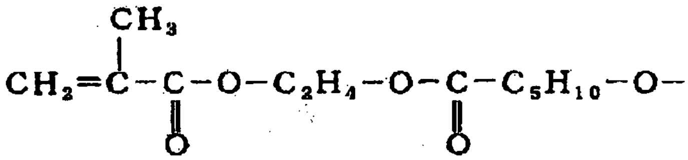 • • • (4)

2. The composite particle according to claim 1, wherein a weight ratio of calcium phosphate is 5 to 30 parts by weight relative to 100 parts by weight of the resin particle, and a weight ratio of calcium carbonate is 3 to 20 parts by weight relative to 100 parts by weight of the resin particle.

3. The composite particle according to claim 1 or 2, wherein an average particle diameter of the composite particle is 1 to 100 μm.

4. The composite particle according to any one of claims 1 to 3, wherein a particle diameter of calcium carbonate is 0.01 to 5 μm.

5. A cosmetic composition comprising: a core-shell-type composite particle comprising a resin particle as a core particle having, formed on the surface thereof, a coating layer comprising amorphous calcium phosphate and calcium carbonate; and a cosmetically acceptable component,
wherein the resin particle contains a polymer of a vinyl monomer and any one of the following components:

a phosphorous acid diester represented by the following general formula (1):

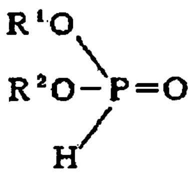 • • • (1)

and a partially esterified phosphoric acid represented by the following general formula (2) or (3):

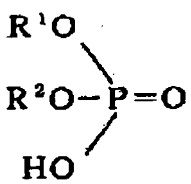 • • • (2) 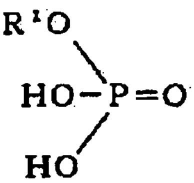 • • • (3)

wherein, $R^1$ and $R^2$ in the general formulae (1) to (3) are each independently a monovalent group formed by removal of one hydrogen atom from an acyclic saturated or unsaturated hydrocarbon having 1 to 14 carbon atoms, or a group represented by the following formula (4):

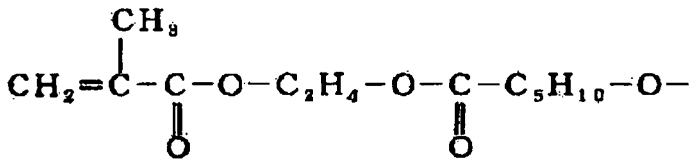

· · · (4)

6. The cosmetic composition according to claim 5, wherein the cosmetic composition comprises the composite particle in an amount of 0.1 to 40% by weight of a total weight of the cosmetic composition.

7. The cosmetic composition according to claim 5 or 6, wherein the cosmetic composition is a make-up cosmetic or a skin care cosmetic.

8. The cosmetic composition according to claim 7, wherein the cosmetic composition is a powder foundation, a W/Si type foundation or O/W type foundation, a loose powder, an eye shadow, or a concealer.

9. The cosmetic composition according to claim 7, wherein the cosmetic composition is a sunscreen.

**Patentansprüche**

1. Verbundpartikel vom Kern-Hülle-Typ, umfassend: ein Harzpartikel als Kernpartikel mit einer, auf dessen Oberfläche ausgebildeten, amorphes Calciumphosphat und Calciumcarbonat umfassenden Überzugsschicht,
wobei das Harzpartikel ein Polymer aus einem Vinylmonomer und eine der folgenden Komponenten enthält:

einen Phosphonsäurediester, wiedergegeben durch die folgende allgemeine Formel (1):

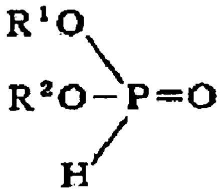

· · · (1)

und eine partiell veresterte Phosphorsäure, wiedergegeben durch die folgende allgemeine Formel (2) oder (3):

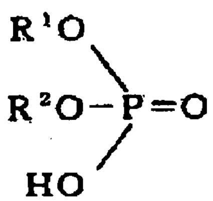

· · · (2)

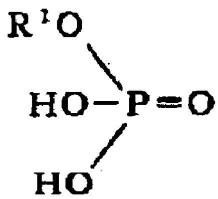

· · · (3)

wobei $R^1$ und $R^2$ in den allgemeinen Formeln (1) bis (3) jeweils unabhängig voneinander ein einwertiger Rest, gebildet durch Entfernung eines Wasserstoffatoms von einem acyclischen gesättigten oder ungesättigten Kohlenwasserstoff mit 1 bis 14 Kohlenstoffatomen, oder ein Rest, wiedergegeben durch die folgende Formel (4), sind:

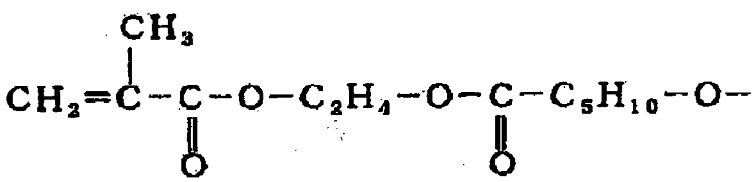

· · · (4)

2. Verbundpartikel nach Anspruch 1, wobei ein Gewichtsanteil von Calciumphosphat 5 bis 30 Gewichtsteile bezüglich

100 Gewichtsteilen des Harzpartikels beträgt und ein Gewichtsanteil von Calciumcarbonat 3 bis 20 Gewichtsteile bezüglich 100 Gewichtsteilen des Harzpartikels beträgt.

**3.** Verbundpartikel nach Anspruch 1 oder 2, wobei ein mittlerer Teilchendurchmesser des Verbundpartikels 1 bis 100 μm beträgt.

**4.** Verbundpartikel nach einem der Ansprüche 1 bis 3, wobei ein Teilchendurchmesser von Calciumcarbonat 0,01 bis 5 μm beträgt.

**5.** Kosmetische Zusammensetzung, umfassend: ein Verbundpartikel vom Kem-Hülle-Typ, umfassend ein Harzpartikel als Kernpartikel mit einer, auf dessen Oberfläche ausgebildeten, amorphes Calciumphosphat und Calciumcarbonat umfassenden Überzugsschicht; und eine kosmetisch akzeptable Komponente,
wobei das Harzpartikel ein Polymer aus einem Vinylmonomer und eine der folgenden Komponenten enthält:

einen Phosphonsäurediester, wiedergegeben durch die folgende allgemeine Formel (1):

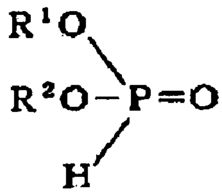

• • • (1)

und eine partiell veresterte Phosphorsäure, wiedergegeben durch die folgende allgemeine Formel (2) oder (3):

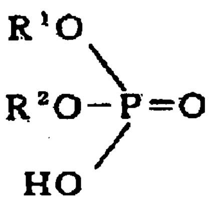

• • • (2)

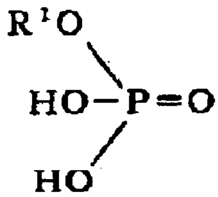

• • • (3)

wobei $R^1$ und $R^2$ in den allgemeinen Formeln (1) bis (3) jeweils unabhängig voneinander ein einwertiger Rest, gebildet durch Entfernung eines Wasserstoffatoms von einem acyclischen gesättigten oder ungesättigten Kohlenwasserstoff mit 1 bis 14 Kohlenstoffatomen, oder ein Rest, wiedergegeben durch die folgende Formel (4), sind:

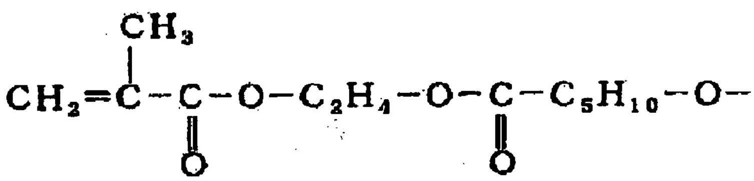

• • • (4)

**6.** Kosmetische Zusammensetzung nach Anspruch 5, wobei die kosmetische Zusammensetzung das Verbundpartikel in einer Menge von 0,1 bis 40 Gew.-% eines Gesamtgewichts der kosmetischen Zusammensetzung umfasst.

**7.** Kosmetische Zusammensetzung nach Anspruch 5 oder 6, wobei die kosmetische Zusammensetzung ein Make-up-Kosmetikum oder ein Hautpflegekosmetikum ist.

**8.** Kosmetische Zusammensetzung nach Anspruch 7, wobei die kosmetische Zusammensetzung eine Pudergrundierung, eine Grundierung vom W/Si-Typ oder eine Grundierung vom O/W-Typ, ein loser Puder, ein Lidschatten oder ein Concealer ist.

**9.** Kosmetische Zusammensetzung nach Anspruch 7, wobei die kosmetische Zusammensetzung ein Sonnenschutzmittel ist.

**Revendications**

**1.** Particule composite de type coeur-écorce comprenant : une particule de résine en tant que particule coeur ayant, formée sur la surface de celle-ci, une couche de revêtement comprenant du phosphate de calcium et du carbonate de calcium amorphes,
dans laquelle la particule de résine contient un polymère d'un monomère vinylique et un quelconque parmi les constituants suivants :

un diester d'acide phosphoreux représenté par la formule générale (I) suivante :

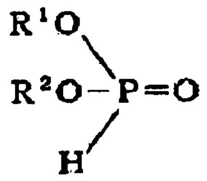
··· (1)

et un acide phosphorique partiellement estérifié représenté par les formules générales (2) ou (3) suivantes :

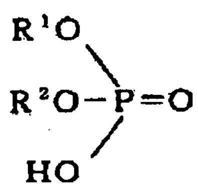
··· (2)

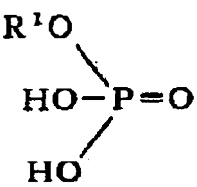
··· (3)

dans lesquelles $R^1$ et $R^2$ dans les formules générales (1) à (3) sont chacun indépendamment un groupe monovalent formé par l'élimination d'un atome d'hydrogène d'un hydrocarbure acyclique saturé ou insaturé ayant de 1 à 14 atomes de carbone, ou un groupe représenté par la formule (4) suivante :

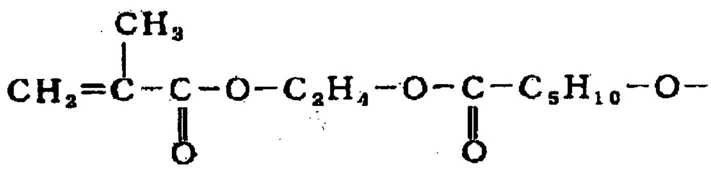
··· (4)

**2.** Particule composite selon la revendication 1, dans laquelle un rapport massique du phosphate de calcium est de 5 à 30 parties en poids par rapport à 100 parties en poids de la particule de résine, et un rapport massique du carbonate de calcium est de 3 à 20 parties en poids par rapport à 100 parties en poids de la particule de résine.

**3.** Particule composite selon la revendication 1 ou 2, dans laquelle un diamètre moyen des particules de la particule composite est de 1 à 100 μm.

**4.** Particule composite selon l'une quelconque des revendications 1 à 3, dans laquelle un diamètre des particules de carbonate de calcium est de 0,01 à 5 μm.

**5.** Composition cosmétique comprenant : une particule composite de type coeur-écorce comprenant une particule de résine en tant que particule coeur ayant, formée sur la surface de celle-ci, une couche de revêtement comprenant du phosphate de calcium et du carbonate de calcium amorphes ; et un constituant cosmétiquement acceptable,
dans laquelle la particule de résine contient un polymère d'un monomère vinylique et un quelconque parmi les constituants suivants :

un diester d'acide phosphoreux représenté par la formule générale (1) suivante :

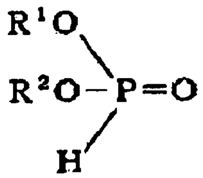

· · · (1)

et un acide phosphorique partiellement estérifié représenté par les formules générales (2) ou (3) suivantes :

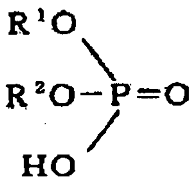

· · · (2)

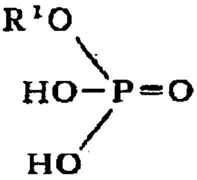

· · · (3)

dans lesquelles $R^1$ et $R^2$ dans les formules générales (1) à (3) sont chacun indépendamment un groupe monovalent formé par l'élimination d'un atome d'hydrogène d'un hydrocarbure acyclique saturé ou insaturé ayant de 1 à 14 atomes de carbone, ou un groupe représenté par la formule (4) suivante :

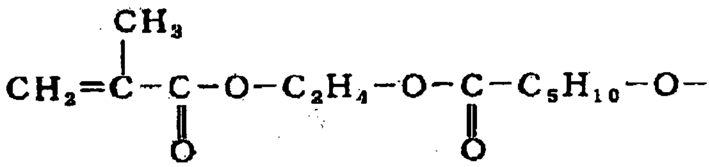

· · · (4)

**6.** Composition cosmétique selon la revendication 5, dans laquelle la composition cosmétique comprend la particule composite dans une quantité de 0,1 à 40 % en poids d'un poids total de la composition cosmétique.

**7.** Composition cosmétique selon la revendication 5 ou 6, dans laquelle la composition cosmétique est un produit cosmétique pour le maquillage ou un produit cosmétique de soin de la peau.

**8.** Composition cosmétique selon la revendication 7, dans laquelle la composition cosmétique est un fond de teint poudre, un fond de teint de type eau/silicone ou un fond de teint de type huile/eau, une poudre libre, un fard à paupières, ou un correcteur.

**9.** Composition cosmétique selon la revendication 7, dans laquelle la composition cosmétique est un écran solaire.

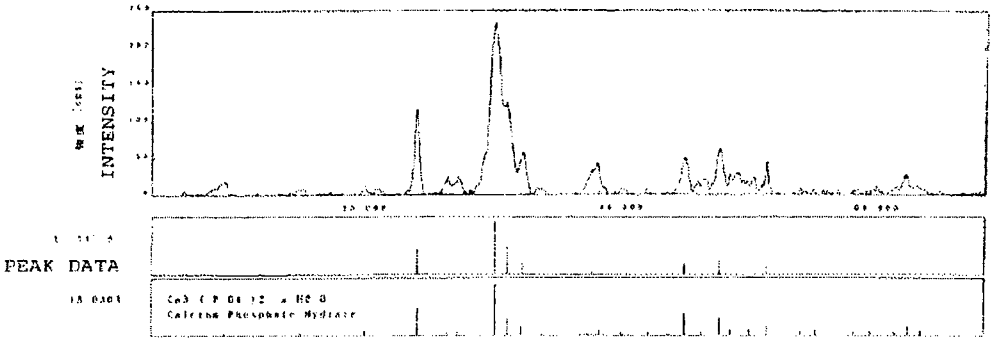


X-RAY DIFFRACTION PATTERN OF AMORPHOUS CALCIUM PHOSPHATE

Fig. 1

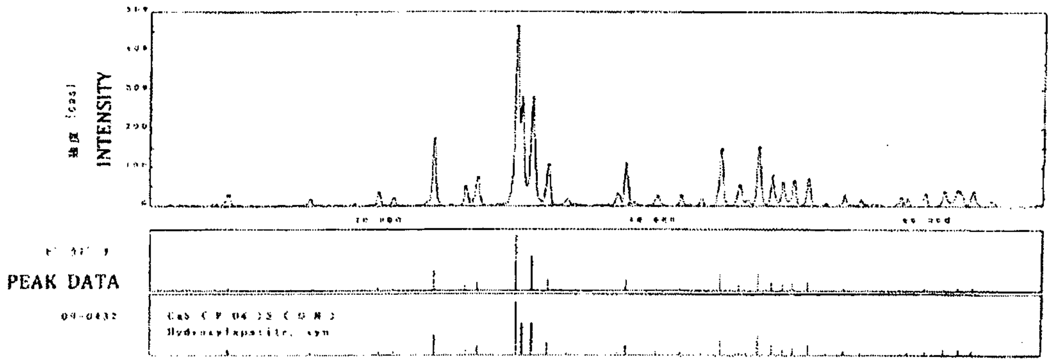


X-RAY DIFFRACTION PATTERN OF CRYSTALLINE CALCIUM PHOSPHATE

Fig. 2

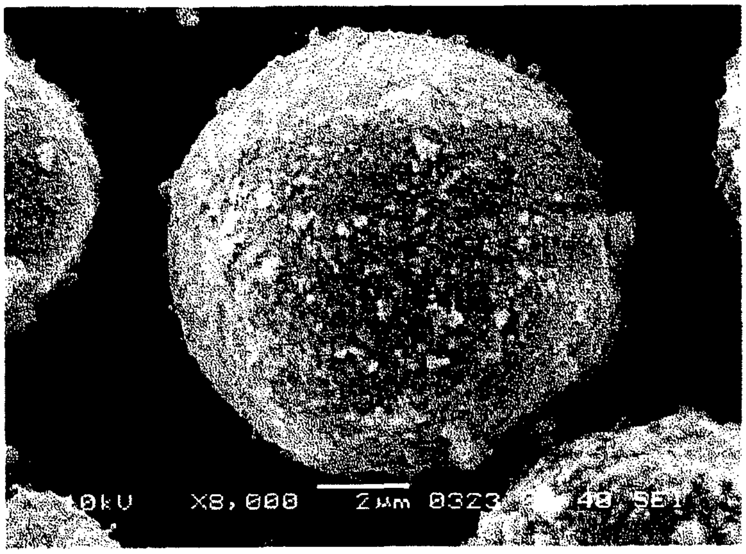


Fig. 3

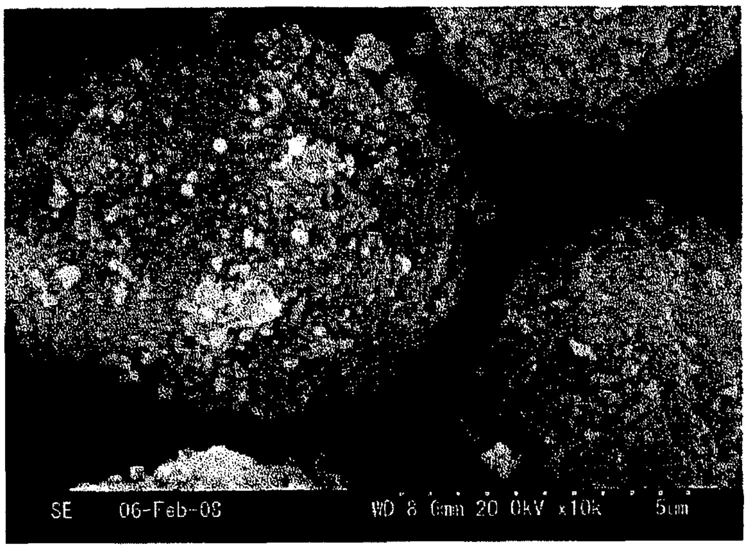


Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2010241785 A
- JP 2243515 A
- JP 2006063062 A
- WO 2009010356 A

### Non-patent literature cited in the description

- *Fragrance Journal,* March 2004, 41-47
- *Fragrance Journal,* March 2004, 41-47
- **SHIN KESHOHINGAKU.** New Cosmetology. Nanzando Co. Ltd, 18 January 2001